(19) Europäisches Patentamt
European Patent Office
Office européen des brevets

(11) Veröffentlichungsnummer: **0 216 880**
**B1**

(12) # EUROPÄISCHE PATENTSCHRIFT

(45) Veröffentlichungstag der Patentschrift: **02.11.88**

(21) Anmeldenummer: **86902376.2**

(22) Anmeldetag: **18.03.86**

(86) Internationale Anmeldenummer: **PCT/EP 86/00156**

(87) Internationale Veröffentlichungsnummer: **WO 86/05484 (25.09.86 Gazette 86/21)**

(51) Int. Cl.⁴: **C 07 C 69/757**, C 07 C 69/14, C 07 C 69/24, C 07 C 13/28, C 07 D 319/06, C 07 D 239/26, C 07 C 43/184, C 07 C 49/00, C 07 C 121/46, C 09 K 19/30, C 09 K 19/34

(54) **CYCLOHEXANDERIVATE.**

(30) Priorität: **22.03.85 DE 3510434**

(43) Veröffentlichungstag der Anmeldung: **08.04.87 Patentblatt 87/15**

(45) Bekanntmachung des Hinweises auf die Patenterteilung: **02.11.88 Patentblatt 88/44**

(84) Benannte Vertragsstaaten: **CH DE FR GB LI NL SE**

(56) Entgegenhaltungen:
**EP-A-0 063 003**
**EP-A-0 117 476**
**EP-A-0 120 627**
**DE-A-2 519 161**

**Chemical Abstracts, vol. 70, no. 15, 14. April 1969, Columbus, Ohio, US, G. Minardi et al.: "Cycloalkylhydrazines", p. 306, abstract no. 67708a**
**Chemische Berichte, vol. 119, no. 2, 4. February 1986, W. Sucrow et al.: "Aliphatische Flüssigkristalle,71), Einige mesogene Tercyclohexalderivate", pp. 387-400**

(73) Patentinhaber: **Merck Patent Gesellschaft mit beschränkter Haftung, Frankfurter Strasse 250, D-6100 Darmstadt (DE)**

(72) Erfinder: **SUCROW, Wolfgang, Hüfferweg 13, D-4790 Paderborn (DE)**
Erfinder: **WOLTER, Herbert, Am Ziegenberg 5, D-4790 Paderborn- Barkhausen (DE)**
Erfinder: **EIDENSCHINK, Rudolf, Kornblumenstrasse 1, D-6115 Münster (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 216 880 B1**

LIBER, STOCKHOLM 1988

**Beschreibung**

Die Erfindung betrifft Cyclohexanderivate der Formel I

$R^1$-$A^1$-$Z^1$-$A^2$-$R^2$ I

worin

$R^1$ und $R^2$ jeweils H, eine Alkylgruppe mit 1 - 10 C-Atomen, worin auch eine oder zwei nicht benachbarte $CH_2$-Gruppen durch O-Atome und/oder -CO-Gruppen und/oder -CO-O-Gruppen und/oder -CH=CH-Gruppen ersetzt sein können, F, Cl, Br, CN oder $R^3$-$A^3$-$Z^2$-,

$A^1$ $A^4$-$Z^0$ -A- oder -A-$Z^0$ -$A^4$-,

A eine unsubstituierte oder in 2-, 3-, 5-und/oder 6-Stellung ein- oder mehrfach durch F und/oder Cl und/oder Br und/oder CN und/oder eine Alkylgruppe oder eine fluorierte Alkylgruppe mit jeweils 1-10 C-Atomen, worin auch eine oder zwei nicht benachbarte $CH_2$-Gruppen durch O-Atome und/oder -CO-Gruppen und/oder -CO-O-Gruppen ersetzt sein können, substituierte trans-1,4-Cyclohexylen- oder 1,4-Cyclohexenylen-Gruppe, die gegebenenfalls auch in 1- und/oder 4-Stellung substituiert sein kann, worin eine oder zwei $CH_2$-Gruppen durch -CO- ersetzt sind, oder

$R^1$-$A^1$- eine in 3-Position durch eine Alkylgruppe mit 1 - 10 C-Atomen, worin auch eine oder zwei nicht benachbarte $CH_2$-Gruppen durch O-Atome und/oder -CO-Gruppen und/oder -CO-O-Gruppen und/oder -CH=CH-Gruppen ersetzt sein können, oder durch -CN substituierte Cyclohexylgruppe, worin auch eine oder zwei nicht benachbarte $CH_2$-Gruppen durch -O- oder -CO- ersetzt sein können,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome und/ oder NO ersetzt sein können, 1,4-Cyclohexylen, worin auch ein oder zwei nicht benachbarte $CH_2$-Gruppen durch O-Atome ersetzt sein können, 1,3-Dithian-2,5-diyl, Piperidin-1,4-diyl, 1,4-Bicyclo (2,2,2)-octylen-, Decahydronaphthalin-2,6-diyl- oder 1,2,3,4-Tetrahydronaphthalin-2,6-diyl-Gruppen, oder -A-

R3 H, eine Alkylgruppe mit 1 - 10 C-Atomen, worin auch eine oder zwei nicht benachbarte $CH_2$-Gruppen durch O-Atome und/oder -CO-Gruppen und/oder -CO-O-Gruppen und/oder -CH=CH-Gruppen ersetzt sein können, F, Cl, Br oder CN bedeutet, mit Ausnahme von 2-(p-Methoxyphenyl)-5-methylcyclohexanon und 2-(p-Methoxyphenyl)-4-methyl-1,3-dioxan, als Komponenten flüssigkristalliner Phasen.

Der Einfachheit halber bedeuten im folgenden Ph eine 1,4-Phenylengruppe, worin auch eine oder mehrere CH-Gruppen durch N ersetzt sein können und die gegebenenfalls auch durch Fluor lateral substituiert sein kann Phe eine 1,4-Phenylengruppe, Cy eine 1,4-cyclohexylengruppe, Dio eine 1,3-Dioxan-2,5-diylgruppe, Bi eine Bicyclo-(2,2,2)-octylengruppe, Pip eine Piperidin-1,4-diylgruppe, Pyr eine Pyrimidin-2,5-diylgruppe, Pyn eine Pyridazin-3,6-diylgruppe, die gegebenenfalls auch als N-oxid vorliegen kann, Dit eine 1,3-Dithian-2,5-diylgruppe und Dec eine Decahydronaphthalin-2,6-diylgruppe.

2-(p-Methoxyphenyl)-5-methylcyclohexanon und 2 (p-Methoxyphenyl)-4-methyl-1,3-dioxan sind in C.A. 70, 67708a und in DE-A-2 519 161 bereits beschrieben. Die Verwendbarkeit dieser oder ähnlicher Verbindungen als Komponenten flüssigkristalliner PHasen war jedoch nicht bekannt.

Ähnliche flüssigkristalline Verbindungen sind z. B. aus der DE-PS-2 636 684 bekannt. Die dort angegebenen Verbindungen enthalten jedoch im Gegensatz zu den vorliegenden keine 1,3-disubstituierten Cyclohexanringe bzw. Cyclohexanonringe.

Die Verbindungen der Formel I können wie ähnliche Verbindungen als Komponenten flüssigkristalliner Dielektrika verwendet werden, insbesondere für Displays, die auf dem Prinzip der verdrillten Zelle, dem Guest-Host-Effekt dem Effekt der Deformation aufgerichteter Phasen dem Prinzip der SSFLC-Technologie (US-P-4 367 924) oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen mit einem für das Zeitmultiplexen günstigen kleinen Wert für den Quotienten $\Delta\varepsilon/\varepsilon_{\|}$, einem hohen Lösungsvermögen für pleochroitische Farbstoffe, sehr kleiner optischer Anisotropie und vergleichsweise niedriger Viskosität herstellbar. Ferner sind mit ihrer Hilfe stabile chirale getiltete smektische flüssigkristalline Phasen mit negativer

dielektrischer Anisotropie herstellbar, die die erforderliche planare Orientierung durch Überlagerung des Ansteuerfeldes mit einem AC-Haltefeld mit kleiner Amplitude ermöglichen (J.M. Geary, SID-Tagung, Orland/Florida, April/Mai 1985, Vortrag 8.3).

Es wurde ferner gefunden, daß die Verbindungen der Formel I als Komponenten chiral getilteter smektischer flüssigkristalliner PHasen vorzüglich geeignet sind. Insbesondere sind mit ihrer Hilfe chemisch besonders stabile chiral getiltete smektische flüssigkristalline Phasen mit günstigen ferroelektrischen Phasenbereichen, insbesondere mit breiten Sc*-Phasenbereichen, negativer oder auch positiver dielektrischer Anisotropie, niedriger optischer Anisotropie, günstiger Pitchhöhe und für derartige Phasen hohen Werten für die spontane Polarisation herstellbar. P ist die spontane Polarisation in $nC/cm^2$.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengestzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken und/oder störende smektische Phasenbereiche in nematischen Mischungen zu unterdrücken. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt, oder daß man zur Herstellung von Estern der Formel I eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von β-Ketoestern der Formel I ein entsprechendes Cyclohexanonderivat in Gegenwart einer Base mit einem entsprechenden Dialkylcarbonat kondensiert,

oder daß man zur Herstellung von Ethern der Formel I eine entsprechende Hydroxyverbindung verethert,

oder daß man zur Herstellung von Cyclohexanonderivaten der Formel I eine entsprechendes Epoxid säurekatalysiert umlagert.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Gegenstand der Erfindung ist ferner eine Flüssigkristallphase mit mindestens zwei flüssigkristallinen Komponenten, dadurch gekennzeichnet, daß sie mindestens eine querpolarisierte 1,4-Cyclohexylenverbindung enthält, worin mindestens eine $CH_2$-Gruppe durch eine -CO-Gruppe ersetzt ist bzw. eine Flüssigkristallphase mit mindestens zwei flüssigkristallinen Komponenten, dadurch gekennzeichnet, daß sie mindestens eine cis-1,3-disubstituierte Cyclohexanverbindung enthält.

Vor- und nachstehend haben $R^1$, $R^2$, $R^3$, $A^1$, $A^2$, $A^3$, $A^4$, A, $Z^1$ und $Z^2$ die angegebende Bedeutung, sofern nicht ausdrücklich etwas anderes vermerkt ist.

Die Verbindungen der Formel I umfassen dementsprechend insbesondere Verbindungen der Teilformeln Ia und Ib (mit zwei Ringen)

$R^1$-A-$A^2$-$R^2$ Ia

$R^1$-A-$Z^1$-$A^2$-$R^2$ Ib

Ic bis Ii (mit drei Ringen),

$R^1$-$A^4$-A-$A^2$-$R^2$ Ic

$R^1$-A-$A^4$-$A^2$-$R^2$ Id

$R^1$-$A^4$-A-$Z^1$-$A^2$-$R^2$ Ie

$R^1$-A-$A^4$-$Z^1$-$A^2$-$R^2$ If

$R^1$-A-$Z^1$-$A^2$-$A^3$-$R^3$ Ig

$R^3$-$A^3$-$Z^2$-A-$Z^1$-$A^2$-$R^2$ Ih

$R^1$-A-$Z^1$-$A^2$-$Z^2$-$A^3$-$R^3$ Ii

sowie Ij bis It (mit vier Ringen)

$R^1$-$A^4$-A-$A^2$-$A^3$-$R^3$ Ij

$R^1$-A-$A^4$-$A^2$-$A^3$-$R^3$ Ik

$R^3$-$A^3$-$Z^2$-$A^4$-A-$A^2$-$R^2$ Il

$R^3$-$A^3$-$A^4$-A-$Z^1$-$A^2$-$R^2$ Im

$R^1$-A-$A^4$-$A^2$-$Z^2$-$A^3$-$R^3$ In

$R^1$-A-$A^4$-$Z^1$-$A^2$-$A^3$-$R^3$ Io

$R^1$-$A^4$-A-$Z^1$-$A^2$-$A^3$-$R^3$ Ip

$R^1$-$A^4$-A-$Z^1$-$A^2$-$Z^2$-$A^3$-$R^3$ Iq

$R^1$-A-$A^4$-$Z^1$-$A^2$-$Z^2$-$A^3$-$R^3$ Ir

$R^3$-$A^3$-$Z^2$-$A^4$-A-$Z^1$-$A^2$-$R^2$ Is

$R^3$-$A^3$-$Z^2$-A-$A^4$-$Z^1$-$A^2$-$R^2$ It

Darunter sind diejenigen der Formeln Ia, Ib, Ic, Id, Ie, If, Ig, Ij und Ik besonders bevorzugt.
Die bevorzugten Verbindungen der Formel Ia umfassen solche der Teilformeln Ia1 bis Ia3:

$R^1$-A-Ph-$R^2$ Ia1

$R^1$-A-Cy-$R^2$ Ia2

$R^1$-A-Bi-$R^2$ Ia3

Darunter sind diejenigen der Teilformeln Ia1 und Ia2 besonders bevorzugt.
Die bevorzugten Verbindungen der Formel Ib umfassen solche der Teilformeln Ib1 bis Ib3:

$R^1$-A-$Z^1$-Ph-$R^2$ Ib1

$R^1$-A-$Z^1$-Cy-$R^2$ Ib2

$R^1$-A-$Z^1$-Bi-$R^2$ Ib3

Darunter sind diejenigen der Teilformeln Ib1 und Ib2, insbesondere diejenigen worin $Z^1$ -CO-O-, O-CO-, -$CH_2$CO-, oder -CO$CH_2$- oder -$CH_2CH_2$- bedeutet, besonders bevorzugt. -$CH_2CH_2$- bedeutet, besonders bevorzugt.
Die bevorzugten Verbindungen der Formel Ic umfassen solche der Teilformeln Ic1 und Ic2:

$R^1$-Cy-A-Cy-$R^2$ Ic1

$R^1$-Cy-A-Ph-$R^2$ Ic2

Die bevorzugten Verbindungen der Formel Id umfassen solche der Teilformeln Id1 bis Id4:

$R^1$-A-Cy-Cy-$R^2$ Id1
$R^1$-A-Ph-Ph-$R^2$ Id2

$R^1$-A-Ph-Cy-$R^2$ Id3
$R^1$-A-Cy-Ph-$R^2$ Id4

Darunter sind diejenigen der Teilformeln Id1 und Id4 besonders bevorzugt.
Die bevorzugten Verbindungen der Formel Ie umfassen solche der Teilformeln Ie1 bis Ie3:

$R^1$-Cy-A-$Z^1$-Cy-$R^2$ Ie1

$R^1$-Cy-A-$Z^1$-Ph-$R^2$ Ie2

$R^1$-Ph-A-$Z^1$-Cy-$R^2$ Ie3

Darunter sind diejenigen der Teilformeln Ie1, insbesondere diejenigen worin $Z^1$ -CO-O-, -O-CO- oder -$CH_2CH_2$-bedeutet, besonders bevorzugt.

Die bevorzugten Verbindungen der Formel If umfassen solche der Teilformeln If1 bis If4:

$R^1$-A-Cy-$Z^1$-Cy-$R^2$ If1

$R^1$-A-Ph-$Z^1$-Ph-$R^2$ If2

$R^1$-A-Ph-$Z^1$-Cy-$R^2$ If3

$R^1$-A-Cy-$Z^1$-Ph-$R^2$ If4

Darunter sind diejenigen der Teilformeln If1, If2 und If3, insbesondere diejenigen worin $Z^1$ -CO-O-, -O-CO oder -$CH_2CH_2$-, insbesondere -CO-O-, bedeutet, besonders bevorzugt.

Die bevorzugten Verbindungen der Formel Ig umfassen solche der Teilformeln Ig1 und Ig3:

$R^1$-A-$Z^1$-Cy-Cy-$R^3$ Ig1

$R^1$-A-$Z^1$-Ph-Cy-$R^2$ Ig2

$R^1$-A-$Z^1$-Ph-Ph-$R^2$ Ig3

Darunter sind diejenigen besonders bevorzugt, worin $Z^1$-O-CO-, -CO-O-, -$CH_2$CO-, -CO$CH_2$- oder -$CH_2CH_2$-bedeutet. Ph-Ph ist vorzugsweise 4,4'-Biphenyl oder -Pyr-Phe-.

Die bevorzugten Verbindungen der Formel Ij umfassen solche der Teilformeln Ij1 und Ij2:

$R^1$-Cy-A-Ph-Ph-$R^3$ Ij1

$R^1$-Cy-A-Ph-Cy-$R^3$ Ij2

Die bevorzugten Verbindungen der Formel Ik umfassen solche der Teilformeln Ik1 und Ik2:

$R^1$-A-Ph-Ph-Cy-$R^3$ Ik1

$R^1$-A-Ph-Cy-Cy-$R^3$ Ik2

In den Verbindungen der vor- und nachstehenden Formeln bedeuten $R^1$, $R^2$ bzw. $R^3$ vorzugsweise Alkyl, ferner Alkoxy- oder eine andere Oxaalkylgruppe.

Weiterhin bevorzugt sind Verbindungen der vor- und nachstehenden Formeln worin einer der Reste $R^1$, $R^2$ bzw. $R^3$ -CO-Alkyl, -O-CO-Alkyl, -CO-O-Alkyl oder CN und der andere Alkyl bedeutet.

In den bevorzugten Verbindungen der vor- und nachstehenden Formeln können die Alkylreste, in denen auch eine $CH_2$-Gruppe (Alkoxy bzw. Oxaalkyl) durch ein O-Atom ersetzt sein kann, geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= 2-Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Methoxy, Octoxy oder Nonoxy.

$A^2$, $A^3$ und $A^4$ sind bevorzugt Cy oder Ph. $Z^1$ und $Z^2$ sind bevorzugt Einfachbindungen, in zweiter Linie bevorzugt -O-CO-, -CO-O-, -$CH_2$CO-, -CO$CH_2$- oder -$CH_2CH_2$-Gruppen.

$Z^0$ ist vorzugsweise eine Einfachbindung.

A ist bevorzugt eine Gruppe ausgewählt aus den Formeln (A) bis (F),

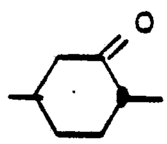

(A)

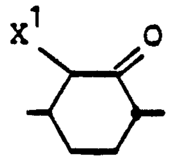

(B)

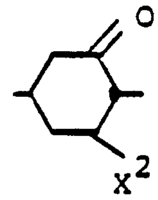

(C)

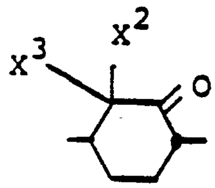

(D)

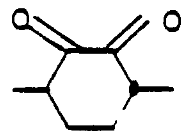

(E)

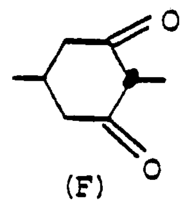

(F)

worin $X^1$, $X^2$ und $X^3$ jeweils unabhängig voneinander F, C1, Br, CN, Alkyl, Alkoxy, Oxaalkyl, Alkanoyl, Alkanoyloxy oder Alkoxycarbonyl mit jeweils 1 bis 10 C-Atomen bedeuten.

A umfaßt auch die Spiegelbilder der Formeln (A) bis (F).

A ist weiterhin bevorzugt eine Gruppe der Formel (G),

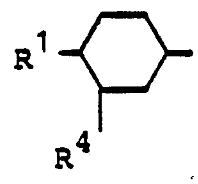

(G)

worin $R^4$ vorzugsweise OH, n-Alkyl, n-Alkoxy, n-Alkanoyloxy, n-Alkoxycarbonyl, CN oder n-Alkanoyl (jeweils vorzugsweise mit 1 bis 10, insbesondere mit 2 bis 7, C-Atomen) bedeutet. $R^1$ bedeutet hier H. Besonders bevorzugt sind Verbindungen der Formel I, worin $R^1$-$A^1$-$Z^1$-einen Rest der Formel (H) in der angegebenen Stereochemie bedeutet:

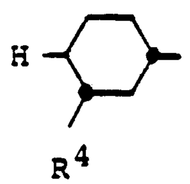

(H)

A ist weiterhin bevorzugt eine Gruppe der Formel (J):

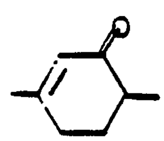

(J)

Die Gruppen der Formeln (A) bis (F) können in 1- oder 4-Stellung einen zusätzlichen axialen Substituenten Q tragen. Q ist vorzugsweise F, CN oder $CH_3$, insbesondere $CH_3$.

Bevorzugte Bedeutungen von $X^1$, $X^2$ und $X^3$ sind F, Cl, CN, $-CH_3$, $-CH_2CH_3$ und $-OCH_3$. Besonders bevorzugt ist CN und $CH_3$ für $X^1$ und F, $CH_3$ und CN für $X^2$ bzw. $X^3$.

Besonders bevorzugt sind die Gruppen (A) und (H).

Ph ist vorzugsweise 1,4-Phenylen, 2-Fluor-1,4-Phenylen, Pyr, Pyn, Pyridin-2,5-diyl oder Pyrazin-2,5-diyl. Besonders bevorzugt sind 1,4-Phenylen und Pyr.

Vorzugsweise enthalten die Verbindungen der Formel I nur einen Ring A.

Verbindungen der vor- und nachstehenden Formeln mit verzweigter Flügelgruppen $R^1$, $R^2$ bzw. $R^3$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Solche Verbindungen sind als

Komponenten smektischer Mischungen mit ferroelektrischen Eigenschaften verwendbar.

Bevorzugte verzweigte Reste sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formel I sowie Ia bis It sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln I1 bis I10:

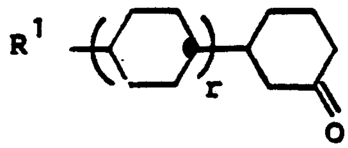 I1

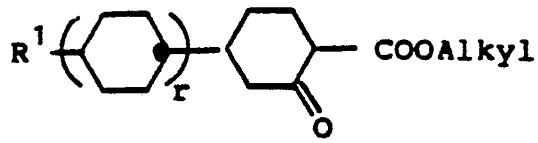 I2

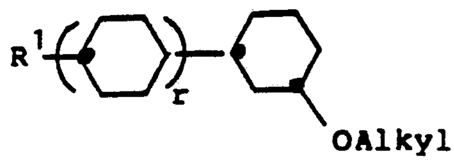 I3

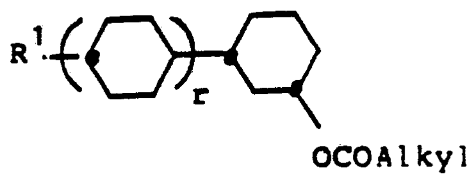 I4

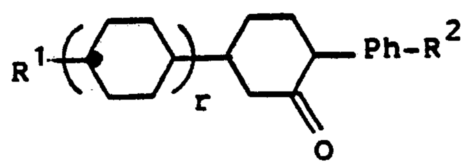 I5

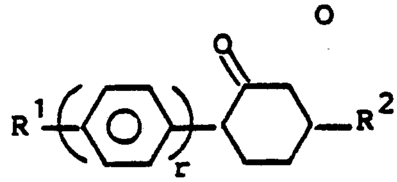 I6

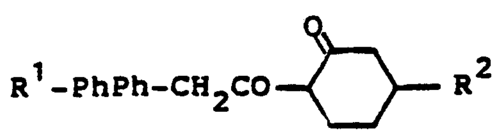 I7

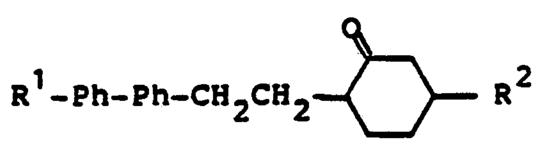 I8

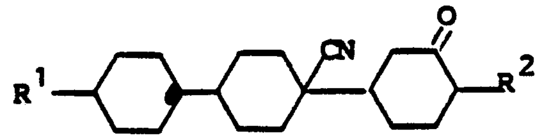

I9

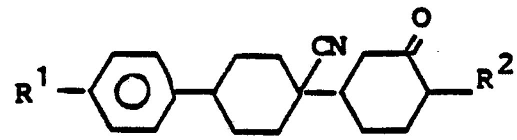

I10

(r ist 0 oder 1)

In den Verbindungen der vorstehenden genannten Fomeln sind diejenigen Stereoisomeren bevorzugt, in denen die Substituenten $R^1$-, $R^1$-$A^4$-, $R^2$-$A^2$-$Z^1$- bzw. $R^2$-$A^2$-$Z^1$-$A^4$-in 1- und 4-Position des Ringes A trans-ständig sind und die äquatoriale Stellung einnehmen, während der gegebenenfalls vorhandene zusätzliche Substituent Q an A in 1-oder 4-Position eine axiale Stellung einnimmt. Diese sind in der Regel stabiler; in vielen Fällen lassen sich die cis-Verbindungen (oder Gemische) durch Behandeln mit einer Base, z. B. mit K-tert.-Butylat in einem inerten Lösungsmittel wie Dimethylsulfoxid, in die trans-Verbindungen umwandeln.

Die Substituenten $X^1$, $X^2$ und $X^3$ in den Gruppen der Formeln (B) und (C) können äquatoriale oder axiale Stellungen einnehmen.

Diejenigen der vorstehend genannten Formeln, die eine oder mehrere Gruppen Dio, Dit, Pip, Pyridin-2,5-diyl und/oder Pyr enthalten, umschließen jeweils die beiden möglichen 2,5-(Dio, Dit, Pyr) bzw. 1,4-Stellungsisomeren (Pip).

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise -CH=CHgruppen in Betracht, ferner z. B. freie oder veresterte Hydroxygruppen, aromatisch gebundene Halogenatome oder Carbonylgruppen. Bevorzugte Ausgangsstoffe für die Reduktion entsprechen der Formel I, können aber an Stelle einer $-CH_2CH_2-$Gruppe eine -CH=CH-Gruppe und/oder an Stelle einer $-CH_2-$Gruppe eine -CO-Gruppe und/oder an Stelle eines H-Atoms eine freie oder eine funktionell (z. B. in Form ihres p-Toluolsulfonats) abgewandelte OH-Gruppe enthalten.

Die Reduktion kann z. B. erfolgen durch katalytische Hydrierung bei Temperaturen zwischen etwa 0° und etwa 200° sowie Drucken zwischen etwa 1 und 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120° oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200° zu den entsprechenden Verbindungen der Formel I, die Alkylgruppen und/oder $-CH_2CH_2-$Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden; so entstehen z. B. aus 1-Cyancyclohexenderivaten die entsprechenden Cyclohexanderivate.

Ester der Formel I können auch durch Veresterung entsprechender Carbonsäuren (oder ihrer

reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischem Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedinungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20°.

Dioxanderivate bzw. Dithianderivate der Formel I werden zweckmäßig durch Reaktion eines entsprechenden Aldehyds (oder eines seiner reaktionsfähigen Derivate) mit einem entsprechenden 1,3-Diol bzw. einem entsprechenden 1,3-Dithiol (oder einem ihrer reaktionsfähigen Derivate) hergestellt, vorzugsweise in Gegenwart eines inerten Lösungsmittels wie Benzol oder Toluol und/oder eines Katalysators, z. B. einer starken Säure wie Schwefelsäure, Benzol- oder p-Toluolsulfonsäure, bei Temperaturen zwischen 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, alle können ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Zur Herstellung von Nitrilen der Formel I können entsprechende Säureamide, z. B. solche in denen an Stelle des Restes X eine $CONH_2$-Gruppe steht, dehydratisiert werden. Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$ $COCl_2$, ferner $P_2O_5$, $P_2S_5$, $AlCl_3$ (z. B. als Doppelverbindungen mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z. B. Basen wie Pyridin oder Triethylamin, aromatische Kohlenwasserstoffe wie Benzol, Toluol, oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z. B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Cyclohexanonderivate der Formel I sind weiterhin durch säurekatalysierte Umlagerungen der

entsprechenden Epoxide nach literaturbekannten Verfahren, z. B. durch Behandeln mit $BF_3$-Etherat, zugänglich. Die Epoxide sind durch Epoxidierung der entsprechenden Cyclohexenderivate nach Standardverfahren erhältlich.

Bevorzugte Cyclohexanderivate der Formel I1,

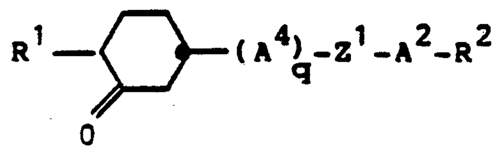

I1

worin $R^1$ vorzugsweise eine Alkylgruppe mit 1 - 10 C-Atomen bedeutet, q 0 oder 1 ist und $A^4$, $Z^1$, $A^2$ und $R^2$ die bei Formel I angegebene Bedeutung haben, sind ferner durch Umsetzung von Verbindungen der Formel I1',

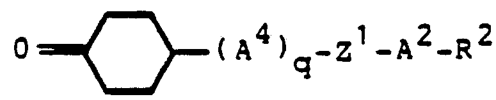

I1'

worin $A^4$, q, $Z^1$, $A^2$ und $R^2$ die oben angegebenen Bedeutungen haben, mit $R^1$-MgX, nachfolgender Wasserabspaltung, Hydroborierung und Oxidation zu I1 analog H. C. Brown und C.P. Garg, J. Am. Chem.Soc. 83, 2951 (1961) zugänglich.

β-Ketoester der Formel I sind beispielsweise nach Standardverfahren durch Kondensation von entsprechenden Cyclohexanonderivaten mit Dialkylcarbonat in Gegenwart einer Base, z. B. KH, erhältlich.

Zur Herstellung von Nitrilen der Formel I können auch entsprechende Chlor- oder Bromverbindungen der Formel I mit einem Cyanid umgesetzt werden, zweckmäßig mit einem Metallcyanid wie NaCN, KCN oder $Cu_2(CN)_2$, z. B. in Gegenwart von Pyridin in einem inerten Lösungsmittel wie DMF oder N-Methylpyrrolidon bei Temperaturen zwischen 20° und 200°.

Die optisch aktiven Verbindungen der Formel I deren Asymmetriezentrum sowohl in den Flügelgruppen $R^1$, $R^2$ bzw. $R^3$ als auch in den Gruppen A bzw. $R^1$-$A^1$- lokalisiert sein kann, erhält man durch den Einsatz entsprechender optisch aktiver Ausgangsmaterialien und/oder durch Trennung der optischen Antipoden mittels Chromatographie nach bekannten Methoden.

Die erfindungsgemäßen Phasen enthalten vorzugsweise mindestens drei, insbesondere mindestens fünf Verbindungen der Formel I. Besonders bevorzugt sind erfindungsgemäße chiral getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Diese weiteren Komponente(n) der chiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Teilformeln Va bis Vp:

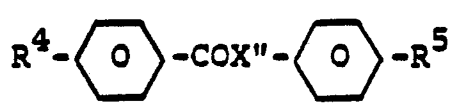

Va

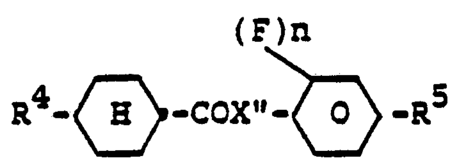

Vb

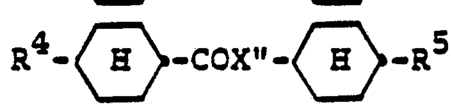

Vc

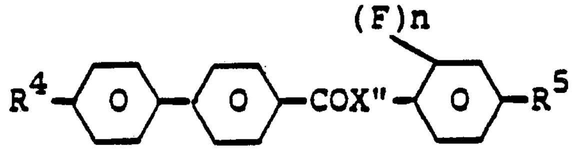 Vd

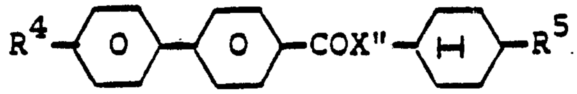 Ve

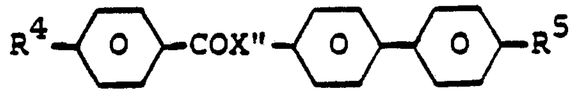 Vf

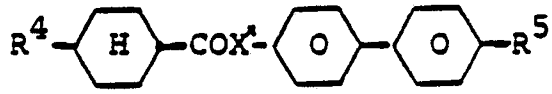 Vg

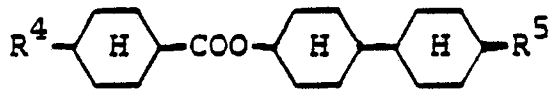 Vh

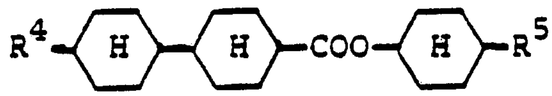 Vi

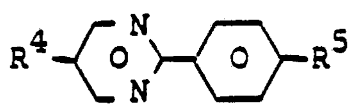 Vj

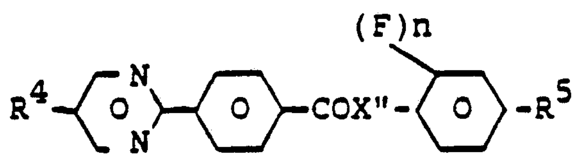 Vk

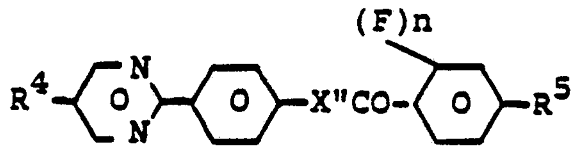 Vl

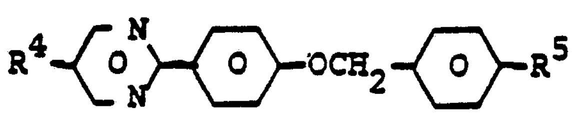 Vm

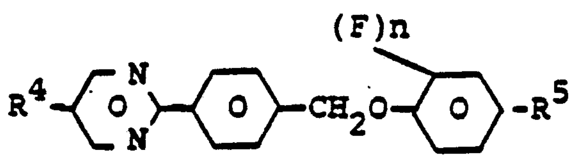 Vn

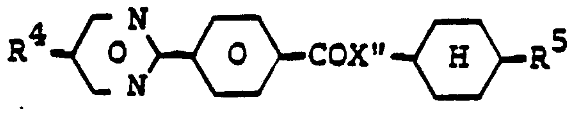 Vo

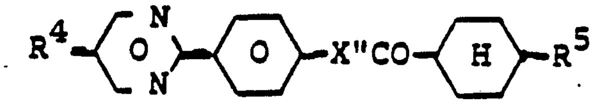 Vp

$R^4$ und $R^5$ sind jeweils vorzugsweise geradkettiges oder einfach verzweigtes Alkyl, Alkoxy, Alkanoyloxy oder Alkoxycarbonyl mit jeweils 3 bis 12 C-Atomen. X" ist O oder S, vorzugsweise O. n ist 0 oder 1.

Besonders bevorzugt sind die Verbindungen der Teilformeln Va, Vb, Vd und Vf, worin $R^4$ und $R^5$ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunkterniedrigung und werden normalerweise den Basismischungen mit nicht mehr als 5 %, vorzugsweise 1 bis 3 %, zugesetzt. $R^4$ und $R^5$ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel

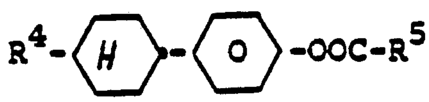

worin $R^4$ und $R^5$ die für Vc, Vh und Vi angegebene bevorzugte Bedeutung haben.

Als weitere Komponenten mit negativer dielektrischer Anisotropie eignen sich weiterhin Verbindungen enthaltend das Strukturelement M, N oder O.

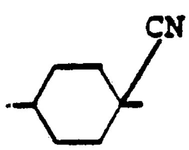 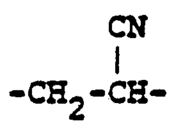 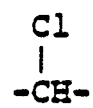

M N O

Bevorzugte Verbindungen dieser Art entsprechen den Formeln VIb und VIc:

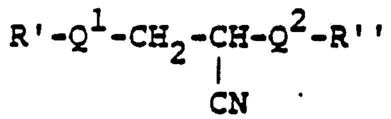 VIb

R'-$Q^3$-$Q^4$-R''' VIc

R' und R'' bedeuten jeweils vorzugsweise geradkettige Alkyl- oder Alkoxy-Gruppen mit jeweils 2 bis 10 C-Atomen.

$Q^1$ und $Q^2$ bedeuten jeweils 1,4-Phenylen, trans-1,4-Cyclohexylen, 4,4'-Biphenylyl, 4-(trans-4-Cyclohexyl)-phenyl, trans, trans-4,4'-Bicyclohexyl oder eine der Gruppen $Q^1$ und $Q^2$ auch eine Einfachbindung.

$Q^3$ und $Q^4$ bedeuten jeweils 1,4-Phenylen, 4,4'-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur

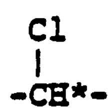

oder

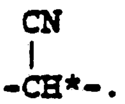

Besonders bevorzugte Verbindungen der Formel VIc sind diejenigen der Formel VIc':

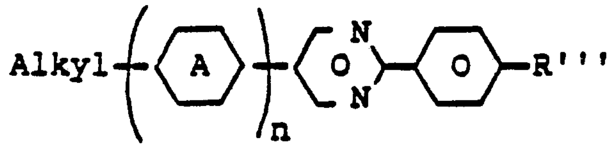

worin A 1,4-Phenylen oder trans-1,4-Cyclohexylen und n 0 oder 1 bedeutet.

Die Verbindungen der Formel I eignen sich auch als Komponenten nematischer flüssigkristalliner Phasen, z. B. zur Vermeidung von reverse twist.

Diese erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Biscyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel I' charakterisieren,

R'-L-G-E-R'' I'

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-Phe-COO- |

oder eine C-C-Einfachbindung,

Y Halogen, vorzugsweise Chlor, oder -CN, und

R' und R'' Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammoniumtetraphenylboranat oder Komplexsalze von Kronenethern (vgl. z. B. I. Haller et al., Mol. Cryst. Liq. Cryst. Band 24, Seiten 249 - 258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität

und/oder der Orientierung der nematischen Phasen zugesetzt werden.

Derartige Substanzen sind z. B. in den DE-OS-2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

**Beispiel 1**

Eine Suspension von 1,7 g $Pd/CaCO_3$ (5 %) in 75 ml Ethanol wird mit einer Lösung von 6,5 g 3-[trans-4-(trans-4-n-propylcyclohexyl)-cyclohexyl]-cyclohex-2-enon [F. 96,5°; erhältlich durch Umsetzung von trans-4-(trans-4-n-Propyl-cyclohexyl)-acetylcyclohexan mit LDA/Ether bei -85°, Zugabe von 1-(Trimethylsilyl)-vinyl-methylketon bei -95°, Zugabe von methanolischer KOH nachdem das Gemisch auf Raumtemperatur erwärmen gelassen wurde und üblicher Aufarbeitung (Kieselgel; Petrolether: Methylenchlorid: Ether = 5 : 3,5 : 1,5)] in 210 ml Ethanol versetzt und 2 Stunden unter Wasserstoff gerührt. Nach dem Entfernen des Katalysators und üblicher Aufarbeitung erhält man 3-[trans-4-(trans-4-n-propylcyclohexyl)-cyclohexyl]-cyclohexanon, F. 51,5°, K. 158,2°.

Analog werden hergestellt:

3-[trans-4-(trans-4-ethylcyclohexyl)-cyclohexyl]-cyclohexanon
3-[trans-4-(trans-4-butylcyclohexyl)-cyclohexyl]-cyclohexanon
3-[trans-4-(trans-4-pentylcyclohexyl)-cyclohexyl]-cyclohexanon
3-[trans-4-(trans-4-hexylcyclohexyl)-cyclohexyl]-cyclohexanon
3-[trans-4-(trans-4-heptylcyclohexyl)-cyclohexyl]-cyclohexanon

3-(trans-4-ethylcyclohexyl)-cyclohexanon
3-(trans-4-propylcyclohexyl)-cyclohexanon
3-(trans-4-butylcyclohexyl)-cyclohexanon
3-(trans-4-pentylcyclohexyl)-cyclohexanon
3-(trans-4-heptylcyclohexyl)-cyclohexanon

3-(trans-4-ethylcyclohexyl)-6-propylcyclohexanon
3-(trans-4-propylcyclohexyl)-6-propylcyclohexanon
3-(trans-4-butylcyclohexyl)-6-propylcyclohexanon
3-(trans-4-pentylcyclohexyl)-6-propylcyclohexanon
3-(trans-4-heptylcyclohexyl)-6-propylcyclohexanon

3-(trans-4-ethylcyclohexyl)-6-butylcyclohexanon
3-(trans-4-propylcyclohexyl)-6-butylcyclohexanon
3-(trans-4-butylcyclohexyl)-6-butylcyclohexanon
3-(trans-4-pentylcyclohexyl)-6-butylcyclohexanon
3-(trans-4-heptylcyclohexyl)-6-butylcyclohexanon

3-(trans-4-ethylcyclohexyl)-6-pentylcyclohexanon
3-(trans-4-propylcyclohexyl)-6-pentylcyclohexanon
3-(trans-4-butylcyclohexyl)-6-pentylcyclohexanon
3-(trans-4-pentylcyclohexyl)-6-pentylcyclohexanon
3-(trans-4-heptylcyclohexyl)-6-pentylcyclohexanon

**Beispiel 2**

Eine Lösung von 2,0 g 3-[trans-4-(trans-4-n-propylcyclohexyl)-cyclohexyl]-cyclohexanon (Beispiel 1) in 40 ml THF wird mit 7,5 mmol KH und 40 ml Dimethylcarbonat versetzt und 2 Stunden gekocht. Nach üblicher Aufarbeitung (Kieselgel; Petrolether: Methylenchlorid: Ether = 6,5 : 3 : 0,5) erhält man 4-[trans-4-(trans-4-n-propylcyclohexyl)-cyclohexyl]-2-oxo-cyclohexancarbonsäuremethylester, der überwiegend in der Enolform vorliegt, F. 154°, K. 192°.

Analog werden hergestellt:

4-[trans-4-(trans-4-propylcyclohexyl)-cyclohexyl]-2-oxo-cyclohexancarbonsäureethylester
4-[trans-4-(trans-4-propylcyclohexyl)-cyclohexyl]-2-oxo-cyclohexancarbonsäurepropylester
4-[trans-4-(trans-4-propylcyclohexyl)-cyclohexyl]-2-oxo-cyclohexancarbonsäurebutylester

4-[trans-4-(trans-4-propylcyclohexyl)-cyclohexyl]-2-oxo-cyclohexancarbonsäurepentylester

4-[trans-4-(trans-4-butylcyclohexyl)-cyclohexyl]-2-oxo-cyclohexancarbonsäuremethylester
4-[trans-4-(trans-4-butylcyclohexyl)-cyclohexyl]-2-oxo-cyclohexancarbonsäureethylester
4-[trans-4-(trans-4-butylcyclohexyl)-cyclohexyl]-2-oxo-cyclohexancarbonsäurepropylester
4-[trans-4-(trans-4-butylcyclohexyl)-cyclohexyl]-2-oxo-cyclohexancarbonsäurebutylester
4-[trans-4-(trans-4-butylcyclohexyl)-cyclohexyl]-2-oxo-cyclohexancarbonsäurepentylester

4-[trans-4-(trans-4-pentylcyclohexyl)-cyclohexyl]-2-oxo-cyclohexancarbonsäuremethylester
4-[trans-4-(trans-4-pentylcyclohexyl)-cyclohexyl]-2-oxo-cyclohexancarbonsäureethylester
4-[trans-4-(trans-4-pentylcyclohexyl)-cyclohexyl]-2-oxo-cyclohexancarbonsäurepropylester
4-[trans-4-(trans-4-pentylcyclohexyl)-cyclohexyl]-2-oxo-cyclohexancarbonsäurebutylester
4-[trans-4-(trans-4-pentylcyclohexyl)-cyclohexyl]-2-oxo-cyclohexancarbonsäurepentylester

4-(trans-4-Propylcyclohexyl)-2-oxo-cyclohexancarbonsäure-methylester
4-(trans-4-Propylcyclohexyl)-2-oxo-cyclohexancarbonsäure-ethylester
4-(trans-4-Propylcyclohexyl)-2-oxo-cyclohexancarbonsäure-propylester
4-(trans-4-Propylcyclohexyl)-2-oxo-cyclohexancarbonsäure-butylester
4-(trans-4-Propylcyclohexyl)-2-oxo-cyclohexancarbonsäure-pentylester

4-(trans-4-Butylcyclohexyl)-2-oxo-cyclohexancarbonsäure-methylester
4-(trans-4-Butylcyclohexyl)-2-oxo-cyclohexancarbonsäure-ethylester
4-(trans-4-Butylcyclohexyl)-2-oxo-cyclohexancarbonsäure-propylester
4-(trans-4-Butylcyclohexyl)-2-oxo-cyclohexancarbonsäure-butylester
4-(trans-4-Butylcyclohexyl)-2-oxo-cyclohexancarbonsäure-pentylester

4-(trans-4-Pentylcyclohexyl)-2-oxo-cyclohexancarbonsäure-methylester
4-(trans-4-Pentylcyclohexyl)-2-oxo-cyclohexancarbonsäure-ethylester
4-(trans-4-Pentylcyclohexyl)-2-oxo-cyclohexancarbonsäure-propylester
4-(trans-4-Pentylcyclohexyl)-2-oxo-cyclohexancarbonsäure-butylester
4-(trans-4-Pentylcyclohexyl)-2-oxo-cyclohexancarbonsäure-pentylester

**Beispiel 3**

Zu einer Lösung von 55 mg Lithium in 150 ml $NH_3$ tropft man eine Lösung von 250 mg 3-[trans-4-(trans-4-n-propyl-cyclohexyl)-cyclohexyl]-cyclohex-2-enon (Synthese beschrieben in Beispiel 1) in 9 ml THF, rührt 2 Stunden, setzt 2 ml Methanol zu, versetzt nochmals mit 250 mg Lithium, rührt 1 Stunde, neutralisiert mit Ammoniumchlorid, setzt 50 ml Methylenchlorid zu, entfernt das $NH_3$ und arbeitet wie üblich auf (Petrolether: Methylenchlorid: Ether = 6,5 : 3 : 0,6). Man erhält r-1-Hydroxy-cis-3-[trans-4-(trans-4-n-propylcyclohexyl)-cyclohexyl]-cyclohexan, F. 217°.

Analog werden hergestellt:

r-1-Hydroxy-cis-3-[trans-4-(trans-4-ethylcyclohexyl)-cyclohexyl]-cyclohexan
r-1-Hydroxy-cis-3-[trans-4-(trans-4-butylcyclohexyl)-cyclohexyl]-cyclohexan
r-1-Hydroxy-cis-3-[trans-4-(trans-4-pentylcyclohexyl)-cyclohexyl]-cyclohexan
r-1-Hydroxy-cis-3-[trans-4-(trans-4-heptylcyclohexyl)-cyclohexyl]-cyclohexan

r-1-Hydroxy-cis-3-(trans-4-ethylcyclohexyl)-cyclohexan
r-1-Hydroxy-cis-3-(trans-4-propylcyclohexyl)-cyclohexan
r-1-Hydroxy-cis-3-(trans-4-butylcyclohexyl)-cyclohexan
r-1-Hydroxy-cis-3-(trans-4-pentylcyclohexyl)-cyclohexan
r-1-Hydroxy-cis-3-(trans-4-heptylcyclohexyl)-cyclohexan

**Beispiel 4**

Zu einer Lösung von 18 mg r-1-Hydroxy-cis-3-[trans-4-(trans-4-n-propylcyclohexyl)-cyclohexyl]-cyclohexan (Beispiel 3) in 1,5 ml Pyridin gibt man 13 mg Buttersäurechlorid, rührt 15 Stunden, arbeitet wie üblich auf und erhält r-1-Butyryloxy-cis-3-[trans-4-(trans-4-n-propylcyclohexyl)-cyclohexyl]-cyclohexan, F. 67,5°, K 91,9°

Analog werden hergestellt:

r-1-Acetoxy-cis-3-[trans-4-(trans-4-propylcyclohexyl)-cyclohexyl]-cyclohexan

r-1-Propionyloxy-cis-3-[trans-4-(trans-4-propylcyclohexyl)-cyclohexyl]-cyclohexan
r-1-Pentanoyloxy-cis-3-[trans-4-(trans-4-propylcyclohexyl)-cyclohexyl]-cyclohexan

r-1-Acetoxy-cis-3-[trans-4-(trans-4-butylcyclohexyl)-cyclohexyl]-cyclohexan
r-1-Propionyloxy-cis-3-[trans-4-(trans-4-butylcyclohexyl)-cyclohexyl]-cyclohexan
r-1-Butyryloxy-cis-3-[trans-4-(trans-4-butylcyclohexyl)-cyclohexyl]-cyclohexan
r-1-Pentanoyloxy-cis-3-[trans-4-(trans-4-butylcyclohexyl)-cyclohexyl]-cyclohexan

r-1-Acetoxy-cis-3-[trans-4-(trans-4-pentylcyclohexyl)-cyclohexyl]-cyclohexan
r-1-Propionyloxy-cis-3-[trans-4-(trans-4-pentylcyclohexyl)-cyclohexyl]-cyclohexan
r-1-Butyryloxy-cis-3-[trans-4-(trans-4-pentylcyclohexyl)-cyclohexyl]-cyclohexan
r-1-Pentanoyloxy-cis-3-[trans-4-(trans-4-pentylcyclohexyl)-cyclohexyl]-cyclohexan

r-1-Acetoxy-cis-3-(trans-4-propylcyclohexyl)-cyclohexan
r-1-Propionyloxy-cis-3-(trans-4-propylcyclohexyl)-cyclohexan
r-1-Butyryloxy-cis-3-(trans-4-propylcyclohexyl)-cyclohexan
r-1-Pentanoyloxy-cis-3-(trans-4-propylcyclohexyl)-cyclohexan

r-1-Acetoxy-cis-3-(trans-4-butylcyclohexyl)-cyclohexan
r-1-Propionyloxy-cis-3-(trans-4-butylcyclohexyl)-cyclohexan
r-1-Butyryloxy-cis-3-(trans-4-butylcyclohexyl)-cyclohexan
r-1-Pentanoyloxy-cis-3-(trans-4-butylcyclohexyl)-cyclohexan

r-1-Acetoxy-cis-3-(trans-4-pentylcyclohexyl)-cyclohexan
r-1-Propionyloxy-cis-3-(trans-4-pentylcyclohexyl)-cyclohexan
r-1-Butyryloxy-cis-3-(trans-4-pentylcyclohexyl)-cyclohexan
r-1-Pentanoyloxy-cis-3-(trans-4-pentylcyclohexyl)-cyclohexan

**Beispiel 5**

Zu einer Lösung von 1,1 g r-1-Hydroxy-cis-3-[trans-4-(trans-4-n-propylcyclohexyl)-cyclohexyl]-cyclohexan (Beispiel 3) in 200 ml THF gibt man 0,5 g 35 %-ige KH-Dispersion und rührt zwei Stunden unter Rückfluß. Dann wird 0,6 g n-Jodbutan zugegeben, zwei Stunden bei Raumtemperatur gerührt, überschüssiges Kaliumhydrid mit Wasser vernichtet und wie üblich aufgearbeitet. Man erhält r-1-n-Butoxy-cis-3-[trans-4-(trans-4-n-propylcyclohexyl)-cyclohexyl)-cyclohexan.

Analog werden hergestellt:

r-1-Methoxy-cis-3-[trans-4-(trans-4-propylcyclohexyl)-cyclohexyl]-cyclohexan
r-1-Ethoxy-cis-3-[trans-4-(trans-4-propylcyclohexyl)-cyclohexyl)-cyclohexan
r-1-Propoxy-cis-3-[trans-4-(trans-4-propylcyclohexyl)-cyclohexyl)-cyclohexan
r-1-Pentoxy-cis-3-[trans-4-(trans-4-propylcyclohexyl)-cyclohexyl]-cyclohexan

r-1-Methoxy-cis-3-[trans-4-(trans-4-butylcyclohexyl)-cyclohexyl]-cyclohexan
r-1-Ethoxy-cis-3-[trans-4-(trans-4-butylcyclohexyl)-cyclohexyl]-cyclohexan
r-1-Propoxy-cis-3-[trans-4-(trans-4-butylcyclohexyl)-cyclohexyl]-cyclohexan
r-1-Butoxy-cis-3-[trans-4-(trans-4-butylcyclohexyl)-cyclohexyl]-cyclohexan
r-1-Pentoxy-cis-3-[trans-4-(trans-4-butylcyclohexyl)-cyclohexyl)-cyclohexan

r-1-Methoxy-cis-3-[trans-4-(trans-4-pentylcyclohexyl)-cyclohexyl]-cyclohexan
r-1-Ethoxy-cis-3-[trans-4-(trans-4-pentylcyclohexyl)-cyclohexyl]-cyclohexan
r-1-Propoxy-cis-3-[trans-4-(trans-4-pentylcyclohexyl)-cyclohexyl]-cyclohexan
r-1-Butoxy-cis-3-[trans-4-(trans-4-pentylcyclohexyl)-cyclohexyl]-cyclohexan
r-1-Pentoxy-cis-3-[trans-4-(trans-4-pentylcyclohexyl)-cyclohexyl]-cyclohexan

r-1-Methoxy-cis-3-(trans-4-propylcyclohexyl)-cyclohexan
r-1-Ethoxy-cis-3-(trans-4-propylcyclohexyl)-cyclohexan
r-1-Propoxy-cis-3-(trans-4-propylcyclohexyl)-cyclohexan
r-1-Butoxy-cis-3-(trans-4-propylcyclohexyl)-cyclohexan
r-1-Pentoxy-cis-3-(trans-4-propylcyclohexyl)-cyclohexan

r-1-Methoxy-cis-3-(trans-4-butylcyclohexyl)-cyclohexan
r-1-Ethoxy-cis-3-(trans-4-butylcyclohexyl)-cyclohexan
r-1-Propoxy-cis-3-(trans-4-butylcyclohexyl)-cyclohexan

r-1-Butoxy-cis-3-(trans-4-butylcyclohexyl)-cyclohexan
r-1-Pentoxy-cis-3-(trans-3-butylcyclohexyl)-cyclohexan

r-1-Methoxy-cis-3-(trans-4-pentylcyclohexyl)-cyclohexan
r-1-Ethoxy-cis-3-(trans-4-pentylcyclohexyl)-cyclohexan
r-1-Propoxy-cis-3-(trans-4-pentylcyclohexyl)-cyclohexan
r-1-Butoxy-cis-3-(trans-4-pentylcyclohexyl)-cyclohexan
r-1-Pentoxy-cis-3-(trans-4-pentylcyclohexyl)-cyclohexan

**Beispiel 6**

Eine Lösung von 20 g p-(4-n-Propylcyclohex-1-en-1-yl)-n-pentylbenzol (DE-OS-3 006 666) in 100 ml Ether wird bei 0° mit 15 g m-Chlorperbenzoesäure versetzt und 16 Stunden gerührt. Das Reaktionsgemisch wird mehrmals mit 10 %-iger Natronlauge extrahiert und die organische Phase mit Natriumsulfat getrocknet. Die erhaltene Epoxidlösung wird mit 40 ml $BF_3$/Ether-Komplex (50 % $BF_3$) versetzt, 5 Minuten gerührt und anschließend auf Eis geschüttet. Nach üblicher Aufarbeitung erhält man trans-2-(p-n-Pentyl-phenyl)-5-n-propylcyclohexanon; Siedepunkt (0,1 Torr) 140°, K. -10°.
Analog werden hergestellt:

trans-2-(p-Pentylphenyl)-5-methylcyclohexanon
trans-2-(p-Pentylphenyl)-5-ethylcyclohexanon
trans-2-(p-Pentylphenyl)-5-butylcyclohexanon
trans-2-(p-Pentylphenyl)-5-pentylcyclohexanon

trans-2-(p-Butylphenyl)-5-methylcyclohexanon
trans-2-(p-Butylphenyl)-5-ethylcyclohexanon
trans-2-(p-Butylphenyl)-5-propylcyclohexanon
trans-2-(p-Butylphenyl)-5-butylcyclohexanon
trans-2-(p-Butylphenyl)-5-pentylcyclohexanon

trans-2-(p-Propylphenyl)-5-methylcyclohexanon
trans-2-(p-Propylphenyl)-5-ethylcyclohexanon
trans-2-(p-Propylphenyl)-5-propylcyclohexanon
trans-2-(p-Propylphenyl)-5-butylcyclohexanon
trans-2-(p-Propylphenyl)-5-pentylcyclohexanon, F. 36°, K. 17° (monotrop)

trans-2-(p-Ethylphenyl)-5-methylcyclohexanon
trans-2-(p-Ethylphenyl)-5-ethylcyclohexanon
trans-2-(p-Ethylphenyl)-5-propylcyclohexanon
trans-2-(p-Ethylphenyl)-5-butylcyclohexanon
trans-2-(p-Ethylphenyl)-5-pentylcyclohexanon

trans-2-(p-Methoxyphenyl)-5-ethylcyclohexanon
trans-2-(p-Methoxyphenyl)-5-propylcyclohexanon
trans-2-(p-Methoxyphenyl)-5-butylcyclohexanon
trans-2-(p-Methoxyphenyl)-5-pentylcyclohexanon

trans-2-(p-Ethoxyphenyl)-5-methylcyclohexanon
trans-2-(p-Ethoxyphenyl)-5-ethylcyclohexanon
trans-2-(p-Ethoxyphenyl)-5-propylcyclohexanon
trans-2-(p-Ethoxyphenyl)-5-butylcyclohexanon
trans-2-(p-Ethoxyphenyl)-5-pentylcyclohexanon

trans-2-(p-Propoxyphenyl)-5-methylcyclohexanon
trans-2-(p-Propoxyphenyl)-5-ethylcyclohexanon
trans-2-(p-Propoxyphenyl)-5-propylcyclohexanon
trans-2-(p-Propoxyphenyl)-5-butylcyclohexanon
trans-2-(p-Propoxyphenyl)-5-pentylcyclohexanon

trans-2-(p-Butoxyphenyl)-5-methylcyclohexanon
trans-2-(p-Butoxyphenyl)-5-ethylcyclohexanon
trans-2-(p-Butoxyphenyl)-5-propylcyclohexanon
trans-2-(p-Butoxyphenyl)-5-butylcyclohexanon

trans-2-(p-Butoxyphenyl)-5-pentylcyclohexanon

trans-2-(p-Pentoxyphenyl)-5-methylcyclohexanon
trans-2-(p-Pentoxyphenyl)-5-ethylcyclohexanon
trans-2-(p-Pentoxyphenyl)-5-propylcyclohexanon
trans-2-(p-Pentoxyphenyl)-5-butylcyclohexanon
trans-2-(p-Pentoxyphenyl)-5-pentylcyclohexanon
trans-2-(p-Hexoxyphenyl)-5-methylcyclohexanon
trans-2-(p-Hexoxyphenyl)-5-ethylcyclohexanon
trans-2-(p-Hexoxyphenyl)-5-propylcyclohexanon
trans-2-(p-Hexoxyphenyl)-5-butylcyclohexanon
trans-2-(p-Hexoxyphenyl)-5-pentylcyclohexanon

trans-2-(p-Heptoxyohenyl)-5-methylcyclohexanon
trans-2-(p-Heptoxyphenyl)-5-ethylcyclohexanon
trans-2-(p-Heptoxyphenyl)-5-propylcyclohexanon
trans-2-(p-Heptoxyphenyl)-5-butylcyclohexanon
trans-2-(p-Heptoxyphenyl)-5-pentylcyclohexanon
trans-2-(p-Octoxyphenyl)-5-methylcyclohexanon
trans-2-(p-Octoxyphenyl)-5-ethylcyclohexanon
trans-2-(p-Octoxyphenyl)-5-propylcyclohexanon
trans-2-(p-Octoxyphenyl)-5-butylcyclohexanon
trans-2-(p-Octoxyphenyl)-5-pentylcyclohexanon
trans-2-(p-Nonoxyphenyl)-5-methylcyclohexanon
trans-2-(p-Nonoxyphenyl)-5-ethylcyclohexanon
trans-2-(p-Nonoxyphenyl)-5-propylcyclohexanon
trans-2-(p-Nonoxyphenyl)-5-butylcyclohexanon
trans-2-(p-Nonoxyphenyl)-5-pentylcyclohexanon

trans-2-(p-Cyanphenyl)-5-methylcyclohexanon
trans-2-(p-Cyanphenyl)-5-ethylcyclohexanon
trans-2-(p-Cyanphenyl)-5-propylcyclohexanon
trans-2-(p-Cyanphenyl)-5-butylcyclohexanon
trans-2-(p-Cyanphenyl)-5-pentylcyclohexanon
trans-2-(4-Pentylbiphenyl-4'-yl)-5-methylcyclohexanon
trans-2-(4-Pentylbiphenyl-4'-yl)-5-ethylcyclohexanon
trans-2-(4-Pentylbiphenyl-4'-yl)-5-propylcyclohexanon
trans-2-(4-Pentylbiphenyl-4'-yl)-5-butylcyclohexanon
trans-2-(4-Pentylbiphenyl-4'-yl)-5-pentylcyclohexanon
trans-2-(4-Heptylbiphenyl-4'-yl)-5-methylcyclohexanon
trans-2-(4-Heptylbiphenyl-4'-yl)-5-ethylcyclohexanon
trans-2-(4-Heptylbiphenyl-4'-yl)-5-propylcyclohexanon
trans-2-(4-Heptylbiphenyl-4'-yl)-5-butylcyclohexanon
trans-2-(4-Heptylbiphenyl-4'-yl)-5-pentylcyclohexanon
trans-2-(4-Methoxybiphenyl-4'-yl)-5-methylcyclohexanon
trans-2-(4-Methoxybiphenyl-4'-yl)-5-ethylcyclohexanon
trans-2-(4-Methoxybiphenyl-4'-yl)-5-propylcyclohexanon
trans-2-(4-Methoxybiphenyl-4'-yl)-5-butylcyclohexanon
trans-2-(4-Methoxybiphenyl-4'-yl)-5-pentylcyclohexanon
trans-2-(4-Ethoxybiphenyl-4'-yl)-5-methylcyclohexanon
trans-2-(4-Ethoxybiphenyl-4'-yl)-5-ethylcyclohexanon
trans-2-(4-Ethoxybiphenyl-4'-yl)-5-propylcyclohexanon
trans-2-(4-Ethoxybiphenyl-4'-yl)-5-butylcyclohexanon
trans-2-(4-Ethoxybiphenyl-4'-yl)-5-pentylcyclohexanon
trans-2-(4-Propoxybiphenyl-4'-yl)-5-methylcyclohexanon
trans-2-(4-Propoxybiphenyl-4'-yl)-5-ethylcyclohexanon
trans-2-(4-Propoxybiphenyl-4'-yl)-5-propylcyclohexanon
trans-2-(4-Propoxybiphenyl-4'-yl)-5-butylcyclohexanon
trans-2-(4-Propoxybiphenyl-4'-yl)-5-pentylcyclohexanon
trans-2-(4-Butoxybiphenyl-4'-yl)-5-methylcyclohexanon
trans-2-(4-Butoxybiehenyl-4'-yl)-5-ethylcyclohexanon
trans-2-(4-Butoxybiphenyl-4'-yl)-5-propylcyclohexanon
trans-2-(4-Butoxybiphenyl-4'-yl)-5-butylcyclohexanon
trans-2-(4-Butoxybiphenyl-4'-yl)-5-pentylcyclohexanon
trans-2-(4-Pentoxybiphenyl-4'-yl)-5-methylcyclohexanon

trans-2-(4-Pentoxybiphenyl-4'-yl)-5-ethylcyclohexanon
trans-2-(4-Pentoxybiphenyl-4'-yl)-5-propylcyclohexanon
trans-2-(4-Pentoxybiphenyl-4'-yl)-5-butylcyclohexanon
trans-2-(4-Pentoxybiphenyl-4'-yl)-5-pentylcyclohexanon
trans-2-(4-Hexoxybiphenyl-4'-yl)-5-methylcyclohexanon
trans-2-(4-Hexoxybiphenyl-4'-yl)-5-ethylcyclohexanon
trans-2-(4-Hexoxybiphenyl-4'-yl)-5-propylcyclohexanon
trans-2-(4-Hexoxybiphenyl-4'-yl)-5-butylcyclohexanon
trans-2-(4-Hexoxybiphenyl-4'-yl)-5-pentylcyclohexanon

trans-2-(4-Heptoxybiphenyl-4'-yl)-5-methylcyclohexanon
trans-2-(4-Heptoxybiphenyl-4'-yl)-5-ethylcyclohexanon
trans-2-(4-Heptoxybiphenyl-4'-yl)-5-propylcyclohexanon
trans-2-(4-Heptoxybiphenyl-4'-yl)-5-butylcyclohexanon
trans-2-(4-Heptoxybiphenyl-4'-yl)-5-pentylcyclohexanon

trans-2-(4-Octoxybiphenyl-4'-yl)-5-methylcyclohexanon
trans-2-(4-Octoxybiphenyl-4'-yl)-5-ethylcyclohexanon
trans-2-(4-Octoxybiphenyl-4'-yl)-5-propylcyclohexanon
trans-2-(4-Octoxybiphenyl-4'-yl)-5-butylcyclohexanon
trans-2-(4-Octoxybiphenyl-4'-yl)-5-pentylcyclohexanon

trans-2-(4-Nonoxybiphenyl-4'-yl)-5-methylcyclohexanon
trans-2-(4-Nonoxybiphenyl-4'-yl)-5-ethylcyclohexanon
trans-2-(4-Nonoxybiphenyl-4'-yl)-5-propylcyclohexanon
trans-2-(4-Nonoxybiphenyl-4'-yl)-5-butylcyclohexanon
trans-2-(4-Nonoxybiphenyl-4'-yl)-5-pentylcyclohexanon

**Beispiel 7**

Eine Lösung von 7,2 g 1-n-Pentyl-5-[trans-4-(trans-4-n-propylcyclohexyl)-cyclohexyl]-cyclohex-1-en [erhältlich durch Umsetzung von 3-[trans-4-(trans-4-n-propylcyclohexyl)-cyclohexyl]-cyclohexanon (Beispiel 1) mit n-Pentylmagnesiumbromid und nachfolgende Wasserabspaltung] in 180 ml Ethanol wird in Gegenwart von 1,2 g Palladium auf Aktivkohle (5 %) bei Raumtemperatur und Normaldruck hydriert. Man erhält nach üblicher Aufarbeitung r-1-n-Pentyl-cis-3-[trans-4-(trans-4-n-propylcyclohexyl)-cyclohexyl]-cyclohexan.

Analog werden hergestellt:

r-1-Pentyl-cis-3-[trans-4-(trans-4-ethylcyclohexyl)-cyclohexyl)-cyclohexan
r-1-Pentyl-cis-3-(trans-4-(trans-4-butylcyclohexyl)-cyclohexyl]-cyclohexan
r-1-Pentyl-cis-3-[trans-4-(trans-4-pentylcyclohexyl)-cyclohexyl)-cyclohexan

r-1-Heptyl-cis-3[trans-4-(trans-4-ethylcyclohexyl)-cyclohexyl]-cyclohexan
r-1-Heptyl-cis-3[trans-4-(trans-4-propylcyclohexyl)-cyclohexyl]-cyclohexan
r-1-Heptyl-cis-3[trans-4-(trans-4-butylcyclohexyl)-cyclohexyl]-cyclohexan
r-1-Heptyl-cis-3[trans-4-(trans-4-pentylcyclohexyl)-cyclohexyl]-cyclohexan
r-1-Heptyl-cis-3[trans-4-(trans-4-heptylcyclohexyl)-cyclohexyl]-cyclohexan

r-1-Butyl-cis-3[trans-4-(trans-4-ethylcyclohexyl)-cyclohexyl]-cyclohexan
r-1-Butyl-cis-3[trans-4-(trans-4-propylcyclohexyl)-cyclohexyl]-cyclohexan
r-1-Butyl-cis-3[trans-4-(trans-4-butylcyclohexyl)-cyclohexyl]-cyclohexan
r-1-Butyl-cis-3[trans-4-(trans-4-pentylcyclohexyl)-cyclohexyl]-cyclohexan
r-1-Butyl-cis-3[trans-4-(trans-4-heptylcyclohexyl)-cyclohexyl]-cyclohexan

r-1-Propyl-cis-3[trans-4-(trans-4-ethylcyclohexyl)-cyclohexyl]-cyclohexan
r-1-Propyl-cis-3[trans-4-(trans-4-propylcyclohexyl)-cyclohexyl]-cyclohexan
r-1-Propyl-cis-3[trans-4-(trans-4-butylcyclohexyl)-cyclohexyl]-cyclohexan
r-1-Propyl-cis-3[trans-4-(trans-4-pentylcyclohexyl)-cyclohexyl]-cyclohexan
r-1-Propyl-cis-3[trans-4-(trans-4-heptylcyclohexyl)-cyclohexyl]-cyclohexan

r-1-Ethyl-cis-3[trans-4-(trans-4-ethylcyclohexyl)-cyclohexyl]-cyclohexan
r-1-Ethyl-cis-3[trans-4-(trans-4-propylcyclohexyl)-cyclohexyl]-cyclohexan
r-1-Ethyl-cis-3[trans-4-(trans-4-butylcyclohexyl)-cyclohexyl]-cyclohexan
r-1-Ethyl-cis-3[trans-4-(trans-4-pentylcyclohexyl)-cyclohexyl]-cyclohexan

r-1-Ethyl-cis-3[trans-4-(trans-4-heptylcyclohexyl)-cyclohexyl]-cyclohexan

r-1-Heptyl-cis-3-(trans-4-ethylcyclohexyl)-cyclohexan
r-1-Heptyl-cis-3-(trans-4-propylcyclohexyl)-cyclohexan
r-1-Heptyl-cis-3-(trans-4-butylcyclohexyl)-cyclohexan
r-1-Heptyl-cis-3-(trans-4-pentylcyclohexyl)-cyclohexan
r-1-Heptyl-cis-3-(trans-4-heptylcyclohexyl)-cyclohexan

r-1-Pentyl-cis-3-(trans-4-ethylcyclohexyl)-cyclohexan
r-1-Pentyl-cis-3-(trans-4-propylcyclohexyl)-cyclohexan
r-1-Pentyl-cis-3-(trans-4-butylcyclohexyl)-cyclohexan
r-1-Pentyl-cis-3-(trans-4-pentylcyclohexyl)-cyclohexan
r-1-Pentyl-cis-3-(trans-4-heptylcyclohexyl)-cyclohexan

r-1-Butyl-cis-3-(trans-4-ethylcyclohexyl)-cyclohexan
r-1-Butyl-cis-3-(trans-4-propylcyclohexyl)-cyclohexan
r-1-Butyl-cis-3-(trans-4-butylcyclohexyl)-cyclohexan
r-1-Butyl-cis-3-(trans-4-pentylcyclohexyl)-cyclohexan
r-1-Butyl-cis-3-(trans-4-heptylcyclohexyl)-cyclohexan

r-1-Propyl-cis-3-(trans-4-ethylcyclohexyl)-cyclohexan
r-1-Propyl-cis-3-(trans-4-propylcyclohexyl)-cyclohexan
r-1-Propyl-cis-3-(trans-4-butylcyclohexyl)-cyclohexan
r-1-Propyl-cis-3-(trans-4-pentylcyclohexyl)-cyclohexan
r-1-Propyl-cis-3-(trans-4-heptylcyclohexyl)-cyclohexan

r-1-Ethyl-cis-3-(trans-4-ethylcyclohexyl)-cyclohexan
r-1-Ethyl-cis-3-(trans-4-propylcyclohexyl)-cyclohexan
r-1-Ethyl-cis-3-(trans-4-butylcyclohexyl)-cyclohexan
r-1-Ethyl-cis-3-(trans-4-pentylcyclohexyl)-cyclohexan
r-1-Ethyl-cis-3-(trans-4-heptylcyclohexyl)-cyclohexan

**Beispiel 8**

Zu einer Lösung von 10,0 g cis-3-(trans-4-n-Pentylcyclohexyl)-cyclohexancarbonsäureamid [erhältlich durch Umsetzung der aus 3-(trans-4-n-Pentylcyclohexyl)-1-brom-cyclohexan (aus dem entsprechenden Alkohol aus Beispiel 3) erhaltenen Grignardverbindung mit $CO_2$ und Umwandlung der Cyclohexancarbonsäure in das Amid] in 180 ml DMF werden bei 40° 50 g Phosphoroxidtrichlorid getropft. Das Reaktionsgemisch wird noch 30 Minuten bei 50° gerührt und nach Abkühlen auf Raumtemperatur auf Eis gegossen. Nach üblicher Aufarbeitung erhält man cis-3-(trans-4-n-Pentylcyclohexyl)-cyclohexancarbonitril.

Analog werden hergestellt:

3-(trans-4-Heptylcyclohexyl)-cyclohexancarbonitril
3-(trans-4-Hexylcyclohexyl)-cyclohexancarbonitril
3-(trans-4-Butylcyclohexyl)-cyclohexancarbonitril
3-(trans-4-Propylcyclohexyl)-cyclohexancarbonitril
3-(trans-4-Ethylcyclohexyl)-cyclohexancarbonitril

3-[trans-4-(trans-4-Heptylcyclohexyl)-cyclohexyl]-cyclohexancarbonitril
3-[trans-4-(trans-4-Hexylcyclohexyl)-cyclohexyl]-cyclohexancarbonitril
3-[trans-4-(trans-4-Pentylcyclohexyl)-cyclohexyl]-cyclohexancarbonitril
3-[trans-4-(trans-4-Propylcyclohexyl)-cyclohexyl]-cyclohexancarbonitril
3-[trans-4-(trans-4-Ethylcyclohexyl)-cyclohexyl]-cyclohexancarbonitril

**Beispiel 9**

Zu einer Lösung von 10,1 g Diisopropylamin in 70 ml THF werden bei -10 ° unter einer Stickstoffatmosphäre nacheinander 62,5 ml Butyllithium in Hexan (1,6 m) und 30,2 g 3-(trans-4-n-Pentylcyclohexyl)-cyclohexancarbonitril (Beispiel 8) gelöst in 40 ml Toluol gegeben und 20 minuten gerührt. Anschließend werden bei -10° 14,2 g Methyljodid zugegeben und weitere 20 Minuten bei Raumtemperatur gerührt. Nach üblicher Aufarbeitung erhält man r-1-Cyan-1-methyl-cis-3-(trans-4-n-Pentylcyclohexyl)-cyclohexan.

Analog werden hergestellt:

r-1-Cyan-1-methyl-cis-3(trans-4-Heptylcyclohexyl)-cyclohexan
r-1-Cyan-1-methyl-cis-3(trans-4-Hexylcyclohexyl)-cyclohexan
r-1-Cyan-1-methyl-cis-3(trans-4-Butylcyclohexyl)-cyclohexan
r-1-Cyan-1-methyl-cis-3(trans-4-Propylcyclohexyl)-cyclohexan
r-1-Cyan-1-methyl-cis-3(trans-4-Ethylcyclohexyl)-cyclohexan

r-1-Cyan-1-methyl-cis-3-[trans-4-(trans-4-Heptylcyclohexyl)-cyclohexyl]-cyclohexan
r-1-Cyan-1-methyl-cis-3-[trans-4-(trans-4-Hexylcyclohexyl)-cyclohexyl]-cyclohexan
r-1-Cyan-1-methyl-ciis-3-[trans-4-(trans-4-Pentylcyclohexyl)-cyclohexyl]-cyclohexan
r-1-Cyan-1-methyl-cis-3-[trans-4-(trans-4-Butylcyclohexyl)-cyclohexyl]-cyclohexan
r-1-Cyan-1-methyl-cis-3-[trans-4-(trans-4-Propylcyclohexyl)-cyclohexyl]-cyclohexan
r-1-Cyan-1-methyl-cis-3-[trans-4-(trans-4-Ethylcyclohexyl)-cyclohexyl]-cyclohexan

Analog erhält man durch Alkylierung mit entsprechenden Alkylhalogeniden:

1-Cyan-1-ethyl-3-[trans-4-(trans-4-Heptylcyclohexyl)-cyclohexyl]-cyclohexan
1-Cyan-1-ethyl-3-[trans-4-(trans-4-Hexylcyclohexyl)-cyclohexyl]-cyclohexan
1-Cyan-1-ethyl-3-[trans-4-(trans-4-Pentylcyclohexyl)-cyclohexyl]-cyclohexan
1-Cyan-1-ethyl-3-[trans-4-(trans-4-Butylcyclohexyl)-cyclohexyl]-cyclohexan
1-Cyan-1-ethyl-3-[trans-4-(trans-4-Propylcyclohexyl)-cyclohexyl]-cyclohexan
1-Cyan-1-ethyl-3-[trans-4-(trans-4-Ethylcyclohexyl)-cyclohexyl]-cyclohexan

1-Cyan-1-propyl-3-[trans-4-(trans-4-Heptylcyclohexyl)-cyclohexyl]-cyclohexan
1-Cyan-1-propyl-3-[trans-4-(trans-4-Hexylcyclohexyl)-cyclohexyl]-cyclohexan
1-Cyan-1-propyl-3-[trans-4-(trans-4-Pentylcyclohexyl)-cyclohexyl]-cyclohexan
1-Cyan-1-propyl-3-[trans-4-(trans-4-Butylcyclohexyl)-cyclohexyl]-cyclohexan
1-Cyan-1-propyl-3-[trans-4-(trans-4-Propylcyclohexyl)-cyclohexyl]-cyclohexan
1-Cyan-1-propyl-3-[trans-4-(trans-4-Ethylcyclohexyl)-cyclohexyl]-cyclohexan

1-Cyan-1-butyl-3-[trans-4-(trans-4-Heptylcyclohexyl)-cyclohexyl]-cyclohexan
1-Cyan-1-butyl-3-[trans-4-(trans-4-Hexylcyclohexyl)-cyclohexyl]-cyclohexan
1-Cyan-1-butyl-3-[trans-4-(trans-4-Pentylcyclohexyl)-cyclohexyl]-cyclohexan
1-Cyan-1-butyl-3-[trans-4-(trans-4-Butylcyclohexyl)-cyclohexyl]-cyclohexan
1-Cyan-1-butyl-3-[trans-4-(trans-4-Propylcyclohexyl)-cyclohexyl]-cyclohexan
1-Cyan-1-butyl-3-[trans-4-(trans-4-Ethylcyclohexyl)-cyclohexyl]-cyclohexan

1-Cyan-1-pentyl-3-[trans-4-(trans-4-Heptylcyclohexyl)-cyclohexyl]-cyclohexan
1-Cyan-1-pentyl-3-[trans-4-(trans-4-Hexylcyclohexyl)-cyclohexyl]-cyclohexan
1-Cyan-1-pentyl-3-[trans-4-(trans-4-Pentylcyclohexyl)-cyclohexyl]-cyclohexan
1-Cyan-1-pentyl-3-[trans-4-(trans-4-Butylcyclohexyl)-cyclohexyl]-cyclohexan
1-Cyan-1-pentyl-3-[trans -4-(trans-4-Propylcyclohexyl)-cyclohexyl]-cyclohexan
1-Cyan-1-pentyl-3-[trans-4-(trans-4-Ethylcyclohexyl)-cyclohexyl]-cyclohexan

1-Cyan-1-heptyl-3-[trans-4-(trans-4-Heptylcyclohexyl)-cyclohexyl)-cyclohexan
1-Cyan-1-heptyl-3-[trans-4-(trans-4-Hexylcyclohexyl)-cyclohexyl]-cyclohexan
1-Cyan-1-heptyl-3-[trans-4-(trans-4-Pentylcyclohexyl)-cyclohexyl]-cyclohexan
1-Cyan-1-heptyl-3-[trans-4-(trans-4-Butylcyclohexyl)-cyclohexyl]-cyclohexan
1-Cyan-1-heptyl-3-[trans-4-(trans-4-Propylcyclohexyl)-cyclohexyl]-cyclohexan
1-Cyan-1-heptyl-3-[trans-4-(trans-4-Ethylcyclohexyl)-cyclohexyl]-cyclohexan

1-Cyan-1-ethyl-3-(trans-4-Heptylcyclohexyl)-cyclohexan
1-Cyan-1-ethyl-3-(trans-4-Hexylcyclohexyl)-cyclohexan
1-Cyan-1-ethyl-3-(trans-4-Pentylcyclohexyl)-cyclohexan
1-Cyan-1-ethyl-3-(trans-4-Butylcyclohexyl)-cyclohexan
1-Cyan-1-ethyl-3-(trans-4-Propylcyclohexyl)-cyclohexan
1-Cyan-1-ethyl-3-(trans-4-Ethylcyclohexyl)-cyclohexan
1-Cyan-1-propyl-3-(trans-4-Heptylcyclohexyl)-cyclohexan
1-Cyan-1-propyl-3-(trans-4-Hexylcyclohexyl)-cyclohexan
1-Cyan-1-propyl-3-(trans-4-Pentylcyclohexyl)-cyclohexan
1-Cyan-1-propyl-3-(trans-4-Butylcyclohexyl)-cyclohexan
1-Cyan-1-propyl-3-(trans-4-Propylcyclohexyl)-cyclohexan
1-Cyan-1-propyl-3-(trans-4-Ethylcyclohexyl)-cyclohexan

1-Cyan-1-butyl-3-(trans-4-Heptylcyclohexyl)-cyclohexan

1-Cyan-1-butyl-3-(trans-4-Hexylcyclohexyl)-cyclohexan
1-Cyan-1-butyl-3-(trans-4-Pentylcyclohexyl)-cyclohexan
1-Cyan-1-butyl-3-(trans-4-Butylcyclohexyl)-cyclohexan
1-Cyan-1-butyl-3-(trans-4-Propylcyclohexyl)-cyclohexan
1-Cyan-1-butyl-3-(trans-4-Ethylcyclohexyl)-cyclohexan

1-Cyan-1-pentyl-3-(trans-4-Heptylcyclohexyl)-cyclohexan
1-Cyan-1-pentyl-3-(trans-4-Hexylcyclohexyl)-cyclohexan
1-Cyan-1-pentyl-3-(trans-4-Pentylcyclohexyl)-cyclohexan
1-Cyan-1-pentyl-3-(trans-4-Butylcyclohexyl)-cyclohexan
1-Cyan-1-pentyl-3-(trans-4-Propylcyclohexyl)-cyclohexan
1-Cyan-1-pentyl-3-(trans-4-Ethylcyclohexyl)-cyclohexan

1-Cyan-1-heptyl-3-(trans-4-Heptylcyclohexyl)-cyclohexan
1-Cyan-1-heptyl-3-(trans-4-Hexylcyclohexyl)-cyclohexan
1-Cyan-1-heptyl-3-(trans-4-Pentylcyclohexyl)-cyclohexan
1-Cyan-1-heptyl-3-(trans-4-Butylcyclohexyl)-cyclohexan
1-Cyan-1-heptyl-3-(trans-4-Propylcyclohexyl)-cyclohexan
1-Cyan-1-heptyl-3-(trans-4-Ethylcyclohexyl)-cyclohexan

**Beispiel 10**

Ein Gemisch von 1,8 g trans-4-n-Pentyl-cyclohexancarboxaldehyd, 1,2 g n-Hexan-1,3-diol, 0,1 g p-Toluolsulfonsäure und 20 ml Toluol wird am Wasserabscheider drei Stunden gekocht, abgekühlt und wie üblich aufgearbeitet. Man erhält r-4-n-Propyl-cis-2-(trans-4-n-pentylcyclohexyl)-1,3-dioxan.

Analog Beispiel 10 erhält man aus den entsprechenden Aldehyden und den entsprechenden 1,3-Diolen:

4-Methyl-2-(trans-4-pentylcyclohexyl)-1,3-dioxan
4-Ethyl-2-(trans-4-pentylcyclohexyl)-1,3-dioxan
4-Butyl-2-(trans-4-pentylcyclohexyl)-1,3-dioxan
4-Pentyl-2-(trans-4-pentylcyclohexyl)-1,3-dioxan
4-Heptyl-2-(trans-4-pentylcyclohexyl)-1,3-dioxan

4-Methyl-2-(trans-4-propylcyclohexyl)-1,3-dioxan
4-Ethyl-2-(trans-4-propylcyclohexyl)-1,3-dioxan
4-Propyl-2-(trans-4-propylcyclohexyl)-1,3-dioxan
4-Butyl-2-(trans-4-propylcyclohexyl)-1,3-dioxan
4-Pentyl-2-(trans-4-propylcyclohexyl)-1,3-dioxan
4-Heptyl-2-(trans-4-propylcyclohexyl)-1,3-dioxan

4-Methyl-2-(p-ethylphenyl)-1,3-dioxan
4-Ethyl-2-(p-ethylphenyl)-1,3-dioxan
4-Propyl-2-(p-ethylphenyl)-1,3-dioxan
4-Butyl-2-(p-ethylphenyl)-1,3-dioxan
4-Pentyl-2-(p-ethylphenyl)-1,3-dioxan
4-Heptyl-2-(p-ethylphenyl)-1,3-dioxan

4-Methyl-2-(p-propylphenyl)-1,3-dioxan
4-Ethyl-2-(p-propylphenyl)-1,3-dioxan
4-Propyl-2-(p-propylphenyl)-1,3-dioxan
4-Butyl-2-(p-propylphenyl)-1,3-dioxan
4-Pentyl-2-(p-propylphenyl)-1,3-dioxan
4-Heptyl-2-(p-propylphenyl)-1,3-dioxan
4-Methyl-2-(p-butylphenyl)-1,3-dioxan
4-Ethyl-2-(p-butylphenyl)-1,3-dioxan
4-Propyl-2-(p-butylphenyl)-1,3-dioxan
4-Butyl-2-(p-butylphenyl)-1,3-dioxan
4-Pentyl-2-(p-butylphenyl)-1,3-dioxan
4-Heptyl-2-(p-butylphenyl)-1,3-dioxan

4-Methyl-2-(p-pentylphenyl)-1,3-dioxan
4-Ethyl-2-(p-pentylphenyl)-1,3-dioxan
4-Propyl-2-(p-pentylphenyl)-1,3-dioxan

4-Butyl-2-(p-pentylphenyl)-1,3-dioxan
4-Pentyl-2-(p-pentylphenyl)-1,3-dioxan
4-Heptyl-2-(p-pentylphenyl)-1,3-dioxan

4-Ethyl-2-(p-methoxyphenyl)-1,3-dioxan
4-Propyl-2-(p-methoxyphenyl)-1,3-dioxan
4-Butyl-2-(p-methoxyphenyl)-1,3-dioxan
4-Pentyl-2-(p-methoxyphenyl)-1,3-dioxan
4-Heptyl-2-(p-methoxyphenyl)-1,3-dioxan

4-Methyl-2-(p-ethoxyphenyl)-1,3-dioxan
4-Ethyl-2-(p-ethoxyphenyl)-1,3-dioxan
4-Propyl-2-(p-ethoxyphenyl)-1,3-dioxan
4-Butyl-2-(p-ethoxyphenyl)-1,3-dioxan
4-Pentyl-2-(p-ethoxyphenyl)-1,3-dioxan
4-Heptyl-2-(p-ethoxyphenyl)-1,3-dioxan

4-Methyl-2-(p-butoxyphenyl)-1,3-dioxan
4-Ethyl-2-(p-butoxyphenyl)-1,3-dioxan
4-Propyl-2-(p-butoxyphenyl)-1,3-dioxan
4-Butyl-2-(p-butoxyphenyl)-1,3-dioxan
4-Pentyl-2-(p-butoxyphenyl)-1,3-dioxan
4-Heptyl-2-(p-butoxyphenyl)-1,3-dioxan

4-Methyl-2-(p-cyanphenyl)-1,3-dioxan
4-Ethyl-2-(p-cyanphenyl)-1,3-dioxan
4-Propyl-2-(p-cyanphenyl)-1,3-dioxan
4-Butyl-2-(p-cyanphenyl)-1,3-dioxan
4-Pentyl-2-(p-cyanphenyl)-1,3-dioxan
4-Heptyl-2-(p-cyanphenyl)-1,3-dioxan

4-Methyl-2-(4-cyan-3-fluorphenyl)-1,3-dioxan
4-Ethyl-2-(4-cyan-3-fluorphenyl)-1,3-dioxan
4-Propyl-2-(4-cyan-3-fluorphenyl)-1,3-dioxan
4-Butyl-2-(4-cyan-3-fluorphenyl)-1,3-dioxan
4-Pentyl-2-(4-cyan-3-fluorphenyl)-1,3-dioxan
4-Heptyl-2-(4-cyan-3-fluorphenyl)-1,3-dioxan

**Beiepiel 11**

Durch Hydrierung von 3-(trans-4-n-Propylcyclohexyl)-6-n-pentyl cyclohex-2-enon [erhältlich nach P. Place et al., Tetrahedron 34, 1931 (1978) durch Umsetzung von 2-Acetyl-butyrolacton mit n-Pentylhalogenid/NaH, nachfolgende Umsetzung mit Bromwasserstoff zum 3-Acetyl-1-brom-n-octan, Überführung in das entsprechende Ethylenketal, Umsetzung der entsprechenden Grignardverbindung mit trans-4-n-Propylcyclohexancarbonitril und nachfolgende Aldolkondensation] analog Beispiel 1 erhält man 2-n-Pentyl-5-(trans-4-n-propylcyclohexyl)-cyclohexanon.

**Beispiel 12**

Eine Lösung von 1,03 g 4-[trans-4-(trans-4-n-Propylcyclohexyl)-cyclohexyl]-2-oxo-cyclohexancarbonsäuremethylester (Beispiel 2) in 20 ml Benzol wird mit 30 ml DMF und 250 mg NaH-Suspension (60 %-ig) versetzt und eine Stunde bei Raumtemperatur unter einer Stickstoffatmosphäre gerührt. Nach Zugabe von 0,43 ml Methyljodid wird weitere 20 Stunden gerührt und wie üblich aufgearbeitet. Man erhält ein Stereoisomerengemisch (79 : 21, wobei das Isomere mit axialer Methylgruppe überwiegt). Nach chromatographischer Auftrennung erhält man trans-4-[trans-4-(trans-4-n-Propylcyclohexyl)-cyclohexyl]-2-oxo-1-methyl-cyclohexan-r-1-carbonsäuremethylester (P. 115°, K. 142°) und cis-4-[trans-4-(trans-4-n-Propylcyclohexyl)-cyclohexyl]-2-oxo-1-methyl-cyclohexan-r-1-carbonsäuremethylester (F. 107°, K. 116°).

Analog werden hergestellt:

trans-4-(trans-4-(trans-4-Propylcyclohexyl)-cyclohexyl]-2-oxo-1-methyl-cyclohexan-r-1-carbonsäureethylester, F. 95°, K. 129°

trans-4-[trans-4-(trans-4-Propylcyclohexyl)-cyclohexyl]-2-oxo-1-methyl-cyclohexan-r-1-carbonsäurepropylester
trans-4-[trans-4-(trans-4-Propylcyclohexyl)-cyclohexyl]-2-oxo-1-methyl-cyclohexan-r-1-carbonsäurebutylester
trans-4-[trans-4-(trans-4-Propylcyclohexyl)-cyclohexyl]-2-oxo-1-methyl-cyclohexan-r-1-carbonsäurepentylester
trans-4-[trans-4-(trans-4-Propylcyclohexyl)-cyclohexyl]-2-oxo-1-methyl-cyclohexan-r-1-carbonsäurehexylester
trans-4-[trans-4-(trans-4-Propylcyclohexyl)-cyclohexyl]-2-oxo-1-methyl-cyclohexan-r-1-carbonsäureheptylester

trans-4-[trans-4-(trans-4-Ethylcyclohexyl)-cyclohexyl]-2-oxo-1-methyl-cyclohexan-r-1-carbonsäuremethylester
trans-4-[trans-4-(trans-4-Ethylcyclohexyl)-cyclohexyl]-2-oxo-1-methyl-cyclohexan-r-1-carbonsäureethylester
trans-4-[trans-4-(trans-4-Ethylcyclohexyl)-cyclohexyl)-2-oxo-1-methyl-cyclohexan-r-1-carbonsäurepropylester
trans-4-[trans-4-(trans-4-Ethylcyclohexyl)-cyclohexyl)-2-oxo-1-methyl-cyclohexan-r-1-carbonsäurebutylester
trans-4-[trans-4-(trans-4-Ethylcyclohexyl)-cyclohexyl]-2-oxo-1-methyl-cyclohexan-r-1-carbonsäurepentylester
trans-4-[trans-4-(trans-4-Ethylcyclohexyl)-cyclohexyl]-2-oxo-1-methyl-cyclohexan-r-1-carbonsäureheptylester

trans-4-(trans-4-(trans-4-Butylcyclohexyl)-cyclohexyl)-2-oxo-1-methyl-cyclohexan-r-1-carbonsäuremethylester
trans-4-[trans-4-(trans-4-Butylcyclohexyl)-cyclohexyl]-2-oxo-1-methyl-cyclohexan-r-1-carbonsäureethylester
trans-4-[trans-4-(trans-4-Butylcyclohexyl)-cyclohexyl]-2-oxo-1-methyl-cyclohexan-r-1-carbonsäurepropylester
trans-4-[trans-4-(trans-4-Butylcyclohexyl)-cyclohexyl]-2-oxo-1-methyl-cyclohexan-r-1-carbonsäurebutyleeter
trans-4-[trans-4-(trans-4-Butylcyclohexyl)-cyclohexyl]-2-oxo-1-methyl-cyclohexan-r-1-carbonsäurepentylester
trans-4-[trans-4-(trans-4-Butylcyclohexyl)-cyclohexyl]-2-oxo-1-methyl-cyclohexan-r-1-carbonsäureheptylester

trans-4-[trans-4-(trans-4-Pentylcyclohexyl)-cyclohexyl]-2-oxo-1-methyl-cyclohexan-r-1-carbonsäuremethylester
trans-4-[trans-4-(trans-4-Pentylcyclohexyl)-cyclohexyl]-2-oxo-1-methyl-cyclohexan-r-1-carbonsäureethylester
trans-4-[trans-4-(trans-4-Pentylcyclohexyl)-cyclohexyl]-2-oxo-1-methyl-cyclohexan-r-1-carbonsäurepropylester
trans-4-[trans-4-(trans-4-Pentylcyclohexyl)-cyclohexyl]-2-oxo-1-methyl-cyclohexan-r-1-carbonsäurebutylester
trans-4-[trans-4-(trans-4-Pentylcyclohexyl)-cyclohexyl]-2-oxo-1-methyl-cyclohexan-r-1-carbonsäurepentylester
trans-4-[trans-4-(trans-4-Pentylcyclohexyl)-cyclohexyl]-2-oxo-1-methyl-cyclohexan-r-1-carbonsäureheptylester

trans-4-[trans-4-(trans-4-Heptylcyclohexyl)-cyclohexyl]-2-oxo-1-methyl-cyclohexan-r-1-carbonsäuremethylester
trans-4-[trans-4-(trans-4-Heptylcyclohexyl)-cyclohexyl]-2-oxo-1-methyl-cyclohexan-r-1-carbonsäureethylester
trans-4-[trans-4-(trans-4-Heptylcyclohexyl)-cyclohexyl]-2-oxo-1-methyl-cyclohexan-r-1-carbonsäurepropylester
trans-4-[trans-4-(trans-4-Heptylcyclohexyl)-cyclohexyl]-2-oxo-1-methyl-cyclohexan-r-1-carbonsäurebutylester
trans-4-[trans-4-(trans-4-Heptylcyclohexyl)-cyclohexyl]-2-oxo-1-methyl-cyclohexan-r-1-carbonsäurepentylester
trans-4-[trans-4-(trans-4-Heptylcyclohexyl)-cyclohexyl]-2-oxo-1-methyl-cyclohexan-r-1-carbonsäureheptylester

cis-4-[trans-4-(trans-4-Propylcyclohexyl)-cyclohexyl)-2-oxo-1-methyl-cyclohexan-r-1-carbonsäureethylester, F. 85°, K. 92°

cis-4-[trans-4-(trans-4-Propylcyclohexyl)-cyclohexyl]-2-oxo-1-methyl-cyclohexan-r-1-carbonsäurepropylester
cis-4-[trans-4-(trans-4-Propylcyclohexyl)-cyclohexyl]-2-oxo-1-methyl-cyclohexan-r-1-carbonsäurebutylester
cis-4-[trans-4-(trans-4-Propylcyclohexyl)-cyclohexyl]-2-oxo-1-methyl-cyclohexan-r-1-carbonsäurepentylester
cis-4-[trans-4-(trans-4-Propylcyclohexyl)-cyclohexyl]-2-oxo-1-methyl-cyclohexan-r-1-carbonsäurehexylester
cis-4-[trans-4-(trans-4-Propylcyclohexyl)-cyclohexyl]-2-oxo-1-methyl-cyclohexan-r-1-carbonsäureheptylester

cis-4-[trans-4-(trans-4-Ethylcyclohexyl)-cyclohexyl]-2-oxo-1-methyl-cyclohexan-r-1-carbonsäuremethylester
cis-4-[trans-4-(trans-4-Ethylcyclohexyl)-cyclohexyl]-2-oxo-1-methyl-cyclohexan-r-1-carbonsäureethylester
cis-4-[trans-4-(trans-4-Ethylcyclohexyl)-cyclohexyl]-2-oxo-1-methyl-cyclohexan-r-1-carbonsäurepropylester
cis-4-[trans-4-(trans-4-Ethylcyclohexyl)-cyclohexyl]-2-oxo-1-methyl-cyclohexan-r-1-carbonsäurebutylester
cis-4-[trans-4-(trans-4-Ethylcyclohexyl)-cyclohexyl]-2-oxo-1-methyl-cyclohexan-r-1-carbonsäurepentylester
cis-4-[trans-4-(trans-4-Ethylcyclohexyl)-cyclohexyl]-2-oxo-1-methyl-cyclohexan-r-1-carbonsäureheptylester

cis-4-[trans-4-(trans-4-Butylcyclohexyl)-cyclohexyl]-2-oxo-1-methyl-cyclohexan-r-1-carbonsäuremethylester
cis-4-[trans-4-(trans-4-Butylcyclohexyl)-cyclohexyl]-2-oxo-1-methyl-cyclohexan-r-1-carbonsäureethylester
cis-4-[trans-4-(trans-4-Butylcyclohexyl)-cyclohexyl]-2-oxo-1-methyl-cyclohexan-r-1-carbonsäurepropylester
cis-4-[trans-4-(trans-4-Butylcyclohexyl)-cyclohexyl]-2-oxo-1-methyl-cyclohexan-r-1-carbonsäurebutylester
cis-4-[trans-4-(trans-4-Butylcyclohexyl)-cyclohexyl]-2-oxo-1-methyl-cyclohexan-r-1-carbonsäurepentylester
cis-4-[trans-4-(trans-4-Butylcyclohexyl)-cyclohexyl)-2-oxo-1-methyl-cyclohexan-r-1-carbonsäureheptylester

cis-4-[trans-4-(trans-4-Pentylcyclohexyl)-cyclohexyl]-2-oxo-1-methyl-cyclohexan-r-1-carbonsäuremethylester
cis-4-[trans-4-(trans-4-Pentylcyclohexyl)-cyclohexyl]-2-oxo-1-methyl-cyclohexan-r-1-carbonsäureethylester
cis-4-[trans-4-(trans-4-Pentylcyclohexyl)-cyclohexyl]-2-oxo-1-methyl-cyclohexan-r-1-carbonsäurepropylester
cis-4-[trans-4-(trans-4-Pentylcyclohexyl)-cyclohexyl]-2-oxo-1-methyl-cyclohexan-r-1-carbonsäurebutylester
cis-4-[trans-4-(trans-4-Pentylcyclohexyl)-cyclohexyl]-2-oxo-1-methyl-cyclohexan-r-1-carbonsäurepentylester
cis-4-[trans-4-(trans-4-Pentylcyclohexyl)-cyclohexyl]-2-oxo-1-methyl-cyclohexan-r-1-carbonsäureheptylester

cis-4-[trans-4-(trans-4-Heptylcyclohexyl)-cyclohexyl]-2-oxo-1-methyl-cyclohexan-r-1-carbonsäuremethylester
cis-4-[trans-4-(trans-4-Heptylcyclohexyl)-cyclohexyl]-2-oxo-1-methyl-cyclohexan-r-1-carbonsäureethylester
cis-4-[trans-4-(trans-4-Heptylcyclohexyl)-cyclohexyl]-2-oxo-1-methyl-cyclohexan-r-1-carbonsäurepropylester
cis-4-(trans-4-(trans-4-Heptylcyclohexyl)-cyclohexyl]-2-oxo-1-methyl-cyclohexan-r-1-carbonsäurebutylester
cis-4-[trans-4-(trans-4-Heptylcyclohexyl)-cyclohexyl]-2-oxo-1-methyl-cyclohexan-r-1-carbonsäurepentylester
cis-4-(trans-4-(trans-4-Heptylcyclohexyl)-cyclohexyl]-2-oxo-1-methyl-cyclohexan-r-1-carbonsäureheptylester

**Beispiel 13**

Eine Lösung von 50 mg 4-[trans-4-(trans-4-n-Propylcyclo-hexyl)-cyclohexyl]-2-oxo-cyclohexancarbonsäuremethylester (Beispiel 2) in 10 ml Dimethoxyethan wird mit 8,5 mg NaH-Suspension (60 %-ig) versetzt und 30 Minuten bei Raumtemperatur unter einer Stickstoffatmosphäre gerührt. Nach Zugabe von einigen Tropfen Acetylchlorid wird noch 1 Stunde bei Raumtemperatur gerührt und wie üblich aufgearbeitet. Man erhält 1-Methoxycarbonyl-4-[trans-4-(trans-4-n-propyl-cyclohexyl)-cyclohexyl]-1-cyclohexen-2-ol-acetat, F. 53°, K. 113°.

Analog werden hergestellt:

1-Ethoxycarbonyl-4-[trans-4-(trans-4-propylcyclohexyl)-cyclohexyl]-1-cyclohexen-2-ol-acetat
1-Propoxycarbonyl-4-[trans-4-(trans-4-propylcyclohexyl)-cyclohexyl]-1-cyclohexen-2-ol-acetat
1-Butoxycarbonyl-4-[trans-4-(trans-4-propylcyclohexyl)-cyclohexyl]-1-cyclohexen-2-ol-acetat
1-Pentoxycarbonyl-4-[trans-4-(trans-4-propylcyclohexyl)-cyclohexyl]-1-cyclohexen-2-ol-acetat

**Beispiel 14**

Ein Gemisch von 5,35 g Diisopropylamin in 45 ml Ether wird bei 0° mit 52,9 mmol Butyllithium versetzt und 45 Minuten gerührt. Nach dem Abkühlen auf -85° wird eine Lösung von 8,28 g trans,trans-4-acetyl-4'-n-propylbicyclohexan (F. 38°, K. 51°, Synthese beschrieben in DE-OS 3 332 690) in 120 ml Ether zugetropft und 2 Stunden bei dieser Temperatur gerührt. Anschließend wird bei -95° eine Lösung von 7,8 g 1-(Trimethylsilyl)-vinyl-methylketon in 70 ml Ether zugetropft und nach 30 Minuten Rühren auf Raumtemperatur erwärmen lassen. Nach Zugabe von 80 ml methanolischer KOH-Lösung (10 %-ig) wird eine Stunde bei Raumtemperatur gerührt, mit verdünnter Salzsäure neutralisiert und wie üblich aufgearbeitet. Man erhält nach chromatographischer Aufreinigung (Kieselgel; Petrolether: Methylenchlorid: Ether = 5 : 3,5 : 1,5) 3-[trans-4-(trans-4-n-propyl-cyclohexyl)-cyclohexyl]-cyclohex-2-enon, F. 96°, K 92° (monotrop).

Analog werden hergestellt:

3-[trans-4-(trans-4-ethylcyclohexyl)-cyclohexyl]-cyclohex-2-enon
3-[trans-4-(trans-4-butylcyclohexyl)-cyclohexyl]-cyclohex-2-enon
3-[trans-4-(trans-4-pentylcyclohexyl)-cyclohexyl]-cyclohex-2-enon
3-[trans-4-(trans-4-hexylcyclohexyl)-cyclohexyl]-cyclohex-2-enon
3-[trans-4-(trans-4-heptylcyclohexyl)-cyclohexyl]-cyclohex-2-enon

3-(trans-4-ethylcyclohexyl)-cyclohex-2-enon
3-(trans-4-propylcyclohexyl)-cyclohex-2-enon
3-(trans-4-butylcyclohexyl)-cyclohex-2-enon
3-(trans-4-pentylcyclohexyl)-cyclohex-2-enon
3-(trans-4-hexylcyclohexyl)-cyclohex-2-enon
3-(trans-4-heptylcyclohexyl)-cyclohex-2-enon
3-(trans-4-methoxycyclohexyl)-cyclohex-2-enon
3-(trans-4-ethoxycyclohexyl)-cyclohex-2-enon
3-(trans-4-propoxycyclohexyl)-cyclohex-2-enon
3-(trans-4-butoxycyclohexyl)-cyclohex-2-enon
3-(trans-4-pentoxycyclohexyl)-cyclohex-2-enon

**Beispiel 15**

Ein Gemisch von 30 g (+)-3-Methylcyclohexanon, 28 g Morpholin, 0,1 g p-Toluolsulfonsäure und 50 ml Toluol wird am Wasserabscheider gekocht. Nach beendeter Wasserabscheidung wird das Lösungsmittel zusammen mit überschüssigem Morpholin abdestilliert und der Rückstand einer Vakuumdestillation unterzogen. Zu einer Lösung von 136 g des erhaltenen Enamins und 9,1 g Triethylamin in 100 ml Toluol gibt man 27,1 g 4'-Pentylbiphenyl-4-yl-essigsäure-chlorid in 20 ml Toluol. Nach 16 h Kochen am Rückfluß wird wie üblich hydrolysiert und aufgearbeitet. Das ölige Rohprodukt wird einer chromatographischen Trennung (Kieselgel/Toluol) unterzogen und aus Ethanol umkristallisiert. Man erhält 2-(4'-Pentylbiphenyl-4-yl-acetyl)-5-methylcyclohexanon, F. 78° (optisch aktiv).

Analog werden hergestellt:

2-(4'-Hexylbiphenyl-4-yl-acetyl)-5-methylcyclohexanon
2-(4'-Hexylbiphenyl-4-yl-acetyl)-5-ethylcyclohexanon
2-(4'-Hexylbiphenyl-4-yl-acetyl)-5-propylcyclohexanon
2-(4'-Hexylbiphenyl-4-yl-acetyl)-5-pentylcyclohexanon
2-(4'-Hexylbiphenyl-4-yl-acetyl)-5-heptylcyclohexanon

2-(4'-Octylbiphenyl-4-yl-acetyl)-5-methylcyclohexanon
2-(4'-Octylbiphenyl-4-yl-acetyl)-5-ethylcyclohexanon
2-(4'-Octylbiphenyl-4-yl-acetyl)-5-propylcyclohexanon
2-(4'-Octylbiphenyl-4-yl-acetyl)-5-pentylcyclohexanon
2-(4'-Octylbiphenyl-4-yl-acetyl)-5-heptylcyclohexanon

2-(4'-Methoxybiphenyl-4-yl-acetyl)-5-methylcyclohexanon
2-(4'-Methoxybiphenyl-4-yl-acetyl)-5-ethylcyclohexanon
2-(4'-Methoxybiphenyl-4-yl-acetyl)-5-propylcyclohexanon
2-(4'-Methoxybiphenyl-4-yl-acetyl)-5-pentylcyclohexanon
2-(4'-Methoxybiphenyl-4-yl-acetyl)-5-heptylcyclohexanon

2-(4'-Ethoxybiphenyl-4-yl-acetyl)-5-methylcyclohexanon
2-(4'-Ethoxybiphenyl-4-yl-acetyl)-5-ethylcyclohexanon
2-(4'-Ethoxybiphenyl-4-yl-acetyl)-5-propylcyclohexanon
2-(4'-Ethoxybiphenyl-4-yl-acetyl)-5-pentylcyclohexanon
2-(4'-Ethoxybiphenyl-4-yl-acetyl)-5-heptylcyclohexanon

2-(4'-Propoxybiphenyl-4-yl-acetyl)-5-methylcyclohexanon
2-(4'-Propoxybiphenyl-4-yl-acetyl)-5-ethylcyclohexanon
2-(4'-Propoxybiphenyl-4-yl-acetyl)-5-propylcyclohexanon
2-(4'-Propoxybiphenyl-4-yl-acetyl)-5-pentylcyclohexanon
2-(4'-Propoxybiphenyl-4-yl-acetyl)-5-heptylcyclohexanon

2-(4'-Butoxybiphenyl-4-yl-acetyl)-5-methylcyclohexanon
2-(4'-Butoxybiphenyl-4-yl-acetyl)-5-ethylcyclohexanon
2-(4'-Butoxybiphenyl-4-yl-acetyl)-5-propylcyclohexanon
2-(4'-Butoxybiphenyl-4-yl-acetyl)-5-pentylcyclohexanon
2-(4'-Butoxybiphenyl-4-yl-acetyl)-5-heptylcyclohexanon

2 (4'-Pentoxybiphenyl-4-yl-acetyl)-5-methylcyclohexanon
2-(4'-Pentoxybiphenyl-4-yl-acetyl)-5-ethylcyclohexanon
2-(4'-Pentoxybiphenyl-4-yl-acetyl)-5-propylcyclohexanon

2-(4'-Pentoxybiphenyl-4-yl-acetyl)-5-pentylcyclohexanon
2-(4'-Pentoxybiphenyl-4-yl-acetyl)-5-heptylcyclohexanon

2-(4'-Hexoxybiphenyl-4-yl-acetyl)-5-methylcyclohexanon
2-(4'-Hexoxybiphenyl-4-yl-acetyl)-5-ethylcyclohexanon
2-(4'-Hexoxybiphenyl-4-yl-acetyl)-5-propylcyclohexanon
2-(4'-Hexoxybiphenyl-4-yl-acetyl)-5-pentylcyclohexanon
2-(4'-Hexoxybiphenyl-4-yl-acetyl)-5-heptylcyclohexanon

2-(4'-Heptoxybiphenyl-4-yl-acetyl)-5-methylcyclohexanon
2-(4'-Heptoxybiphenyl-4-yl-acetyl)-5-ethylcyclohexanon
2-(4'-Heptoxybiphenyl-4-yl-acetyl)-5-propylcyclohexanon
2-(4'-Heptoxybiphenyl-4-yl-acetyl)-5-pentylcyclohexanon
2-(4'-Heptoxybiphenyl-4-yl-acetyl)-5-heptylcyclohexanon

2-(4'-Octoxybiphenyl-4-yl-acetyl)-5-methylcyclohexanon
2-(4'-Octoxybiphenyl-4-yl-acetyl)-5-ethylcyclohexanon
2-(4'-Octoxybiphenyl-4-yl-acetyl)-5-propylcyclohexanon
2-(4'-Octoxybiphenyl-4-yl-acetyl)-5-pentylcyclohexanon
2-(4'-Octoxybiphenyl-4-yl-acetyl)-5-heptylcyclohexanon

2-(4'-Nonoxybiphenyl-4-yl-acetyl)-5-methylcyclohexanon
2-(4'-Nonoxybiphenyl-4-yl-acetyl)-5-ethylcyclohexanon
2-(4'-Nonoxybiphenyl-4-yl-acetyl)-5-propylcyclohexanon
2-(4'-Nonoxybiphenyl-4-yl-acetyl)-5-pentylcyclohexanon
2-(4'-Nonoxybiphenyl-4-yl-acetyl)-5-heptylcyclohexanon

2-[p-(5-Hexylpyrimidin-2-yl)-phenylacetyl]-5-methylcyclohexanon
2-[p-(5-Hexylpyrimidin-2-yl)-phenylacetyl]-5-ethylcyclohexanon
2-[p-(5-Hexylpyrimidin-2-yl)-phenylacetyl]-5-propylcyclohexanon
2-[p-(5-Hexylpyrpyrimidin-2-yl)-phenylacetyl]-5-pentylcyclohexanon
2-[p-(5-Hexylpyrimidin-2-yl)-phenylacetyl]-5-heptylcyclohexanon

2-[p-(5-Octylpyrimidin-2-yl)-phenylacetyl]-5-methylcyclohexanon
2-[p-(5-Octylpyrimidin-2-yl)-phenylacetyl]-5-ethylcyclohexanon
2-[p-(5-Octylpyrimidin-2-yl)-phenylacetyl]-5-propylcyclohexanon
2-[p-(5-Octylpyrimidin-2-yl)-phenylacetyl]-5-pentylcyclohexanon
2-[p-(5-Octylpyrimidin-2-yl)-phenylacetyl]-5-heptylcyclohexanon

2-[p-(5-Nonylpyrimidin-2-yl)-phenylacetyl]-5-methylcyclohexanon
2-[p-(5-Nonylpyrimidin-2-yl)-phenylacetyl]-5-ethylcyclohexanon
2-[p-(5-Nonylpyrimidin-2-yl)-phenylacetyl]-5-propylcyclohexanon
2-[p-(5-Nonylpyrimidin-2-yl)-phenyl]-5-pentylcyclohexanon
2-[p-(5-Nonylpyrimidin-2-yl)-phenylacetyl]-5-heptylcyclohexanon

2-[p-(5-Decylpyrimidin-2-yl)-phenylacetyl]-5-metylcyclohexanon
2-[p-(5-Decylpyrimidin-2-yl)-phenylacetyl]-5-ethylcyclohexanon
2-[p-(5-Decylpyrimidin-2-yl)-phenylacetyl]-5-propylcyclohexanon
2-[p-(5-Decylpyrimidin-2-yl)-phenylacetyl]-5-pentylcyclohexanon
2-[p-(5-Decylpyrimidin-2-yl)-phenylacetyl]-5-heptylcyclohexanon

2-[p-(5-Heptylpyrimidin-2-yl)-phenylacetyl]-5-methylcyclohexanon
2-[p-(5-Heptylpyrimidin-2-yl)-phenylacetyl]-5-ethylcyclohexanon
2-[p-(5-Heptylpyrimidin-2-yl)-phenylacetyl]-5-propylcyclohexanon
2-[p-(5-Heptylpyrimidin-2-yl)-phenylacetyl]-5-pentylcyclohexanon
2-[p-(5-Heptylpyrimidin-2-yl)-phenylacetyl]-5-heptylcyclohexanon
2-[p-(5-Pentylpyrimidin-2-yl)-phenylacetyl]-5-methylcyclohexanon
2-[p-(5-Pentylpyrimidin-2-yl)-phenylacetyl]-5-ethylcyclohexanon
2-[p-(5-Pentylpyrimidin-2-yl)-phenylacetyl]-5-propylcyclohexanon
2-[p-(5-Pentylpyrimidin-2-yl)-phenylacetyl]-5-pentylcyclohexanon
2-[p-(5-Pentylpyrimidin-2-yl)-phenylacetyl]-5-heptylcyclohexanon

2-[p-(5-Hexylpyridin-2-yl)-phenylacetyl]-5-methylcyclohexanon
2-[p-(5-Hexylpyridin-2-yl)-phenylacetyl]-5-ethylcyclohexanon
2-[p-(5-Hexylpyridin-2-yl)-phenylacetyl]-5-propylcyclohexanon

2-[p-(5-Hexylpyridin-2-yl)-phenylacetyl]-5-pentylcyclohexanon
2-[p-(5-Hexylpyridin-2-yl)-phenylacetyl]-5-heptylcyclohexanon

2-[p-(trans-4-Pentylcyclohexyl)-phenylacetyl]-5-methylcyclohexanon
2-[p-(trans-4-Pentylcyclohexyl)-phenylacetyl]-5-ethylcyclohexanon
2-[p-(trans-4-Pentylcyclohexyl)-phenylacetyl]-5-propylcyclohexanon
2-[p-(trans-4-Pentylcyclohexyl)-phenylacetyl]-5-pentylcyclohexanon
2-[p-(trans-4-Pentylcyclohexyl)-phenylacetyl]-5-heptylcyclohexanon

2-[p-(trans-4-Heptylcyclohexyl)-phenylacetyl]-5-methylcyclohexanon
2-[p-(trans-4-Heptylcyclohexyl)-phenylacetyl]-5-ethylcyclohexanon
2-[p-(trans-4-Heptylcyclohexyl)-phenylacetyl]-5-propylcyclohexanon
2-[p-(trans-4-Heptylcyclohexyl)-phenylacetyl]-5-pentylcyclohexanon
2-[p-(trans-4-Heptylcyclohexyl)-phenylacetyl]-5-heptylcyclohexanon

2-[p-(trans-4-Propylcyclohexyl)-phenylacetyl]-5-methylcyclohexanon
2-[p-(trans-4-Propylcyclohexyl)-phenylacetyl]-5-ethylcyclohexanon
2-[p-(trans-4-Propylcyclohexyl)-phenylacetyl]-5-propylcyclohexanon
2-[p-(trans-4-Propylcyclohexyl)-phenylacetyl]-5-pentylcyclohexanon
2-[p-(trans-4-Propylcyclohexyl)-phenylacetyl]-5-heptylcyclohexanon

2-(trans-trans-4-Propylcyclohexylcyclohex-4'-yl-acetyl)-5-methylcyclohexanon
2-(trans-trans-4-Propylcyclohexylcyclohex-4'-yl-acetyl)-5-ethylcyclohexanon
2-(trans-trans-4-Propylcyclohexylcyclohex-4'-yl-acetyl)-5-propylcyclohexanon
2-(trans-trans-4-Propylcyclohexylcyclohex-4'-yl-acetyl)-5-pentylcyclohexanon
2-(trans-trans-4-Propylcyclohexylcyclohex-4'-yl-acetyl)-5-heptylcyclohexanon

2-(trans-trans-4-Pentylcyclohexylcyclohex-4'-yl-acetyl)-5-methylcyclohexanon
2-(trans-trans-4-Pentylcyclohexylcyclohex-4'-yl-acetyl)-5-ethylcyclohexanon
2-(trans-trans-4-Pentylcyclohexylcyclohex-4'-yl-acetyl)-5-propylcyclohexanon
2-(trans-trans-4-Pentylcyclohexylcyclohex-4'-yl-acetyl)-5-pentylcyclohexanon
2-(trans-trans-4-Pentylcyclohexylxyxlohex-4'-yl-acetyl)-5-heptylcyclohexanon

2-(trans-trans-4-Heptylcyclohexylcyclohex-4'-yl-acetyl)-5-methylcyclohexanon
2-(trans-trans-4-Heptylcyclohexylcyclohex-4'-yl-acetyl)-5-ethylcyclchexanon
2-(trans-trans-4-Heptylcyclohexylcyclohex-4'-yl-acetyl)-5-propylcyclohexanon
2-(trans-trans-4-Heptylcyclohexylcyclohex-4'-yl-acetyl)-5-pentylcyclohexanon
2-(trans-trans-4-Heptylcyclohexylcyclohex-4'-yl-acetyl)-5-heptylcyclohexanon

2-[trans-4-(p-Ethoxyphenyl)-cyclohexylacetyl]-5-methylcyclohexanon
2-[trans-4-(p-Ethoxyphenyl)-cyclohexylacetyl]-5-ethylcyclohexanon
2-[trans-4-(p-Ethoxyphenyl)-cyclohexylacetyl]-5-propylcyclohexanon
2-[trans-4-(p-Ethoxyphenyl)-cyclohexylacetyl]-5-pentylcyclohexanon
2-[trans-4-(p-Ethoxyphenyl)-cyclohexylacetyl]-5-heptylcyclohexanon

2-[trans-4-(p-Butoxyphenyl)-cyclohexylacetyl]-5-methylcyclohexanon
2-[trans-4-(p-Butoxyphenyl)-cyclohexylacetyl]-5-ethylcyclohexanon
2-[trans-4-(p-Butoxyphenyl)-cyclohexylacetyl]-5-propylcyclohexanon
2-[trans-4-(p-Butoxyphenyl)-cyclchexylacetyl]-5-pentylcyclohexanon
2-[trans-4-(p-Butoxyphenyl)-cyclohexylacetyl]-5-heptylcyclohexancn

2-[trans-4-(p-Hexoxyphenyl)-cyclohexylacetyl]-5-methylcyclohexanon
2-[trans-4-(p-Hexoxyphenyl)-cyclohexylacetyl]-5-ethylcyclohexanon
2-[trans-4-(p-Hexoxyphenyl)-cyclchexylacetyl]-5-propylcyclohexanon
2-[trans-4-(p-Hexoxyphenyl)-cyclohexylacetyl]-5-pentylcyclohexanon
2-[trans-4-(p-Hexoxyphenyl)-cyclohexylacetyl]-5-heptylcyclohexanon

2-[trans-4-(p-Octoxyphenyl)-cyclohexylacetyl]-5-methylcyclohexanon
2-[trans-4-(p-Octoxyphenyl)-cyclohexylacetyl]-5-ethylcyclohexanon
2-[trans-4-(p-Octoxyphenyl)-cyclohexylacetyl]-5-propylcyclohexanon
2-[trans-4-(p-Octoxyphenyl)-cyclohexylacetyl]-5-pentylcyclohexanon
2-[trans-4-(p-Octoxyphenyl)-cyclohexylacetyl]-5-heptylcyclohexanon

2-[trans-4-(p-Nonoxyphenyl)-cyclohexylacetyl]-5-methylcyclohexanon
2-[trans-4-(p-Nonoxyphenyl)-cyclohexylacetyl]-5-ethylcyclohexanon

2-[trans-4-(p-Nonoxyphenyl)-cyclohexylacetyl]-5-propylcyclohexanon
2-[trans-4-(p-Nonoxyphenyl)-cyclohexylacetyl]-5-pentylcyclohexanon
2-[trans-4-(p-Nonoxyphenyl)-cyclchexylacetyl]-5-heptylcyclchexanon

2-(p-Ethoxyphenylacetyl)-5-methylcyclohexanon
2-(p-Ethoxyphenylacetyl)-5-ethylcyclohexanon
2-(p-Ethoxyphenylacetyl)-5-propylcyclohexanon
2-(p-Ethoxyphenylacetyl)-5-pentylcyclohexanon
2-(p-Ethoxyphenylacetyl)-5-heptylcyclohexanon

2-(p-Butoxyphenylacetyl)-5-methylcyclohexanon
2-(p-Butoxyphenylacetyl)-5-ethylcyclohexanon
2-(p-Butoxyphenylacetyl)-5-propylcyclohexanon
2-(p-Butoxyphenylacetyl)-5-pentylcyclohexanon
2-(p-Butoxyphenylacetyl)-5-heptylcyclohexanon

2-(p-Hexoxyphenylacetyl)-5-methylcyclohexanon
2-(p-Hexoxyphenylacetyl)-5-ethylcyclohexanon
2-(p-Hexoxyphenylacetyl)-5-propylcyclohexanon
2-(p-Hexoxyphenylacetyl)-5-pentylcyclohexanon
2-(p-Hexoxyphenylacetyl)-5-heptylcyclohexanon

2-(p-Nonoxyphenylacetyl)-5-methylcyclohexanon
2-(p-Nonoxyphenylacetyl)-5-ethylcyclohexanon
2-(p-Nonoxyphenylacetyl)-5-propylcyclohexanon
2-(p-Nonoxyphenylacetyl)-5-pentylcyclohexanon
2-(p-Nonoxyphenylacetyl)-5-heptylcyclohexanon

**Beispiel 16**

Zu einer Lösung von 26,3 g trans-trans-4'-Pentyl-bicyclohexyl-4-carbonitril in 100 ml THF wird bei -78° unter Stickstoffatmosphäre eine Lösung von Lithiumdiisopropylamid - hergestellt aus 60 ml THF, 13,2 g Diisopropylamin und 75 ml einer 1,6molaren Lösung von n-Butyllithium in Hexan - langsam zugefügt. Nach einstündigem Rühren werden langsam 11,5 g 2-Cyclohexen-1-on zugetropft. Nach einer weiteren Stunden Rühren wird das Reaktionsgemisch auf Raumtemperatur erwärmt und hydrolysiert. Nach üblicher Aufarbeitung, chromatographischer Aufreinigung (Kieselgel/ Toluol) und Umkristallisation aus Aceton erhält man r-1-Cyan-1-(3-oxocyclohexyl)-cis-4-(trans-4-pentylcyclohexyl)-cyclohexan, F. 120°.

Analog werden hergestellt:

r-1-Cyan-1-(3-oxo-4-propylcyclohexyl)-cis-4-(trans-4-pentylcyclohexyl)-cyclohexan
r-1-Cyan-1-(3-oxocyclohexyl)-cis-4-(p-propylphenyl)-cyclohexan
r-1-Cyan-1-(3-oxocyclohexyl)-cis-4-(p-butylphenyl)-cyclohexan
r-1-Cyan-1-(3-oxocyclohexyl)-cis-4-(p-pentylphenyl)-cyclohexan
r-1-Cyan-1-(3-oxocyclohexyl)-cis-4-(p-heptylphenyl)-cyclohexan
r-1-Cyan-1-(3-oxocyclohexyl)-cis-4-(p-octylphenyl)-cyclohexan
r-1-Cyan-1-(3-oxocyclohexyl)-cis-4-(p-methoxyphenyl)-cyclohexan
r-1-Cyan-1-(3-oxocyclohexyl)-cis-4-(p-butoxyphenyl)-cyclohexan
r-1-Cyan-1-(3-oxocyclohexyl)-cis-4-(p-pentoxyphenyl)-cyclohexan
r-1-Cyan-1-(3-oxocyclohexyl)-cis-4-(p-hexoxyphenyl)-cyclohexan

r-1-Cyan-1-(3-oxocyclohexyl)-cis-4-(4-propylbiphenyl-4'-yl)-cyclohexan
r-1-Cyan-1-(3-oxocyclohexyl)-cis-4-(4-butylbiphenyl-4'-yl)-cyclohexan
r-1-Cyan-1-(3-oxocyclohexyl)-cis-4-(4-pentylbiphenyl-4'-yl)-cyclohexan
r-1-Cyan-1-(3-oxocyclohexyl)-cis-4-(4-heptylbiphenyl-4'-yl)-cyclohexan
r-1-Cyan-1-(3-oxocyclohexyl)-cis-4-(4-octylbiphenyl-4'-yl)-cyclohexan
r-1-Cyan-1-(5-oxocyclohexyl)-cis-4-(4-methoxybiphenyl-4'-yl)-cyclohexan
r-1-Cyan-1-(3-oxocyclohexyl)-cis-4-(4-butoxybiphenyl-4'-yl)-cyclohexan
r-1-Cyan-1-(3-oxocyclohexyl)-cis-4-(4-pentoxybiphenyl-4'-yl)-cyclohexan
r-1-Cyan-1-(3-oxocyclohexyl)-cis-4-(4-hexoxybiphenyl-4'-yl)-cyclohexan

r-1-Cyan-1-(3-oxo-4-propylcyclohexyl)-cis-4-(4-propylbiphenyl-4'-yl)-cyclohexan
r-1-Cyan-1-(3-oxo-4-propylcyclohexyl)-cis-4-(4-butylbiphenyl-4'-yl)-cyclohexan
r-1-Cyan-1-(3-oxo-4-propylcyclohexyl)-cis-4-(4-pentylbiphenyl-4'-yl)-cyclohexan

r-1-Cyan-1-(3-oxo-4-propylcyclohexyl)-cis-4-(4-heptylbiphenyl-4'-yl)-cyclohexan
r-1-Cyan-1-(3-oxo-4-propylcyclohexyl)-cis-4-(4-octylbiphenyl-4'-yl)-cyclohexan
r-1-Cyan-1-(3-oxo-4-propylcyclohexyl)-cis-4-(4-methoxybiphenyl-4'-yl)-cyclohexan
r-1-Cyan-1-(3-oxo-4-propylcyclohexyl)-cis-4-(4-butoxybiphenyl-4'-yl)-cyclohexan
r-1-Cyan-1-(3-oxo-4-propylcyclohexyl)-cis-4-(4-pentoxybiphenyl-4'-yl)-cyclohexan
r-1-Cyan-1-(3-oxo-4-propylcyclohexyl)-cis-4-(4-hexoxybiphenyl-4'-yl)-cyclohexan

Es folgen Beispiele für FK-Phasen mit einem Gehalt an mindestens einer Verbindung der Formel I:

**Beispiel A**

Man stellt eine flüssigkristalline Phase her, bestehend aus

17 % p-(trans-4-Propylcyclohexyl)-benzonitril,
22 % p-(trans-4-Butylcyclohexyl)-benzonitril,
24 % p-(trans-4-Pentylcyclohexyl)-benzonitril,
14 % p-(trans-4-Heptylcyclohexyl)-benzonitril,
18 % 4-Ethyl-4'-(trans-4-pentylcyclohexyl)-biphenyl und
5 % trans-2-(p-Propylphenyl)-5-pentylcyclohexanon.

**Beispiel B**

Man stellt eine flüssigkristalline Phase her, bestehend aus

16 % trans-1-(p-Ethoxyphenyl)-4-propylcyclohexan,
12 % trans-1-(p-Butoxyphenyl)-4-propylcyclohexan,
16 % p-(trans-4-Propylcyclohexyl)-benzonitril,
23 % p-(trans-4-Pentylcyclohexyl)-benzonitril,
15 % p-(trans-4-Heptylcyclohexyl)-benzonitril und
18 % 3-[trans-4-(trans-4-Propylcyclohexyl)-cyclohexyl]-cyclohexanon.

**Beispiel C**

Man stellt eine flüssigkristalline Phase her, bestehend aus

16 % trans-1-(p-Ethoxyphenyl)-4-propylcyclohexan,
12 % trans-1-(p-Butoxyphenyl)-4-propylcyclohexan,
16 % p-(trans-4-Propylcyclohexyl)-benzonitril,
23 % p-(trans-4-Pentylcyclohexyl)-benzonitril,
15 % p-(trans-4-Heptylcyclohexyl)-benzonitril und
18 % r-1-Butyryloxy-cis-3-[trans-4-(trans-4-propylcyclo-hexyl)-cyclohexyl]-cyclohexan.

**Beispiel D**

Eine ferroelektrische flüssigkristalline Phase bestehend aus

3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
27 % 2-p-Nonyloxyphenyl-5:nonylpyrimidin,
26 % r-1-cyan-cis-4-(4'-butyloxybiphenyl-4-yl)-1-octylcyclohexan,
13 % r-1-cyan-cis-4-(4'-hexylbiphenyl-4-yl)-1-heptylcyclohexan,
5 % r-1-cyan-1-(trans-4-pentylcyclohexyl)-cis-4-(trans-4-pentylcyclohexyl)-cyclohexan und
10 % r-1-cyan-1-(3-oxocyclohexyl)-cis-4-(trans-4-pentylcyclohexyl)-cyclohexan (optisch aktiv) zeigt K/$S_C^*$ -7°, $S_C^*$/$S_A^*$ 54°, $S_A^*$ / Ch 79°, Ch/I 90°.

**Patentansprüche**

1. Cyclohexanderivate der Formel I

$R^1$-$A^1$-$Z^1$-$A^2$-$R^2$ I

worin

$R^1$ und $R^2$ jeweils H, eine Alkylgruppe mit 1 - 10 C-Atomen, worin auch eine oder zwei nicht benachbarte $CH_2$-Gruppen durch O-Atome und/ oder -CO-Gruppen und/oder -CO-O-Gruppen und/oder -CH=CH-Gruppen ersetzt sein können, F, Cl, Br, CN oder $R^3$-$A^3$-$Z^2$-,

$A^1$ -A-, $A^4$-$Z^o$-A- oder -A-$Z^o$ -$A^4$-,

A eine unsubstituierte oder in 2-, 3-, 5-und/oder 6-Stellung ein- oder mehrfach durch F und/oder Cl und/oder Br und/oder CN und/oder eine Alkylgruppe oder eine fluorierte Alkylgruppe mit jeweils 1-10 C-Atomen, worin auch eine oder zwei nicht benachbarte $CH_2$-Gruppen durch O-Atome und/oder -CO-Gruppen und/oder -CO-O-Gruppen ersetzt sein können, substituierte trans-1,4-Cyclohexylen- oder 1,4-Cyclohexenylen-Gruppe, die gegebenenfalls auch in 1- und/oder 4-Stellung substituiert sein kann, worin eine oder zwei $CH_2$-Gruppen durch -CO- ersetzt sind, oder

$R^1$-$A^1$- eine in 3-Position durch eine Alkylgruppe mit 1 - 10 C-Atomen, worin auch eine oder zwei nicht benachbarte $CH_2$-Gruppen durch O-Atome und/oder -CO-Gruppen und/oder -CO-O-Gruppen und/oder -CH=CH-Gruppen ersetzt sein können, oder durch -CN substituierte Cyclohexylgruppe, worin auch eine oder zwei nicht benachbarte $CH_2$-Gruppen durch -O- oder -CO- ersetzt sein können,

$A^2$, $A^3$ und $A^4$
jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome und/ oder NO ersetzt sein können, 1,4-Cyclohexylen, worin auch ein oder zwei nicht benachbarte $CH_2$-Gruppen durch O-Atome ersetzt sein können, 1,3-Dithian-2,5-diyl, Piperidin-1,4-diyl, 1,4-Bicyclo (2,2,2)-octylen-, Decahydronaphthalin-2,6-diyl- oder 1,2,3,4-Tetrahydronaphthalin-2,6-diyl-Gruppen, oder -A-

$Z^o$, $Z^1$ und $Z^2$
jeweils -CO-O-, -O-CO-, -$CH_2CH_2$-,-$OCH_2$-, -$CH_2O$- -$CH_2CO$-, -$COCH_2$-, -$CH_2CHCN$; -$CHCNCH_2$-, oder eine Einfachbindung, und

$R^3$ H, eine Alkylgruppe mit 1 - 10 C-Atomen, worin auch eine oder zwei nicht benachbarte $CH_2$-Gruppen durch O-Atome und/oder -CO-Gruppen und/oder -CO-O-Gruppen und/oder -CH=CH-Gruppen ersetzt sein können, F, Cl, Br oder CN bedeutet,
mit Ausnahme von 2-(p-Methoxyphenyl)-5-methylcyclohexanon und 2-(p-Methoxyphenyl)-4-methyl-1,3-dioxan.

2. Verfahren zur Herstellung von Cyclohexanderivaten der Formel I nach Anspruch 1, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder daß man zur Herstellung von Estern der Fomel I eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von β-Ketoestern der Fomel I ein entsprechendes Cyclohexanonderivat in Gegenwart einer Base mit einem entsprechenden Dialkylcarbonat kondensiert,

oder daß man zur Herstellung von Ethern der Formel I eine entsprechende Hydroxyverbindung verethert,

oder daß man zur Herstellung von Cyclohexanonderivaten der Formel I ein entsprechendes Epoxid säurekatalysiert umlagert.

3. Verwendung der Verbindungen der Formel I nach Anspruch 1 als Komponenten flüssigkristalliner Phasen.

4. Flüssigkristallphase mit mindestens zwei flüssigkristallinen Komponenten, dadurch gekennzeichnet, daß sie mindestens eine querpolarisierte 1,4-Cyclohexylenverbindung enthält, worin mindestens eine $CH_2$-Gruppe durch eine -CO-Gruppe ersetzt ist.

5. Flüssigkristallphase mit mindestens zwei flüssigkristallinen Komponenten, dadurch gekennzeichnet, daß sie mindestens eine cis-1,3-disubstituierte Cyclohexanverbindung enthält.

6. Flüssigkristalline Phase nach Anspruch 4 oder 5, dadurch gekennzeichnet, daß mindestens eine Komponente eine Verbindung der Formel I nach Anspruch 1 ist.

7. Flüssigkristallanzeigeelement, dadurch gekennzeichnet, daß es eine Phase nach Anspruch 4, 5 oder 6 enthält.

8. Elektrooptisches Anzeigeelement nach Anspruch 7, dadurch gekennzeichnet, daß es als Dielektrikum eine

Phase nach Anspruch 4, 5 oder 6 enthält.

## Claims

1. Cyclohexane derivatives of the formula I

$R^1$-$A^1$-$Z^1$-$A^2$-$R^2$ I

wherein $R^1$ and $R^2$ are each H or an alkyl group which has 1 - 10 C atoms and in which one or two non-adjacent $CH^2$ groups can also be replaced by O atoms and/or -CO- groups and/or -CO-O- groups and/or -CH=CH- groups, or are F, Cl, Br, CN or $R^3$-$A^3$-$Z^2$-,

$A^1$ is -A-, $A^4$-Z°-A- or -A-Z°-$A^4$-,

A is a trans-1,4-cyclohexylene or 1,4-cyclohexenylene group which is unsubstituted or is monosubstituted or polysubstituted in the 2-, 3-, 5- and/or 6-position by F and/or Cl and/or Br and/or CN and/or an alkyl group or a fluorinated alkyl group each of which has 1 - 10 C atoms and in which one or two non-adjacent $CH_2$ groups can also be replaced by O atoms and/or -CO- groups and/or -CO-O-groups, which trans-1,4-cyclohexylene or 1,4-cyclohexenylene group can optionally also be substituted in the 1-position and/or 4-position and one or two $CH_2$ groups therein can be replaced by -CO-, or

$R^1$-$A^1$- is a cyclohexyl group which is substituted in the 3-position by an alkyl group which has 1 - 10 C atoms and in which one or two non-adjacent $CH_2$ groups can also be replaced by O atoms and/or -CO- groups and/or -CO-O-groups and/or -CH=CH- groups, or is substituted in the 3-position by CN, in which cyclohexyl group one or two non-adjacent $CH_2$ groups can also be replaced by -O- or -CO-, $A^2$, $A^3$ and $A^4$ are each 1,4-phenylene which is unsubstituted or substituted by one or two F and/or Cl atoms and/or $CH_3$ groups and/or CN groups and in which one or two CH groups can also be replaced by N atoms and/or NO, or are 1,4-cyclohexylene wherein one or two non-adjacent $CH_2$ groups can also be replaced by O atoms, or are 1,3-dithiane-2,5-diyl, piperidine-1,4-diyl, 1,4-bicyclo(2,2,2)-octylene, decahydronaphtalene-2,6-diyl or 1,2,3,4-tetra-hydro-napthalene-2,6-diyl groups or are -A-,

Z°, $Z^1$ and $Z^2$ are each -CO-O-, -O-CO-, -$CH_2CH_2$-, $OCH_2$-, -$CH_2O$-, -$CH_2CO$-, -$COCH_2$-, -$CH_2CHCN$-, -$CHCNCH_2$- or a single bond, and

$R^3$ is H or an alkyl group which has 1 -10 C atoms and in which one or two non-adjacent $CH_2$ groups can also be replaced by O atoms and/or -CO-groups and/or -CO-O- groups and/or -CH=CH- groups, or is F, Cl, Br or CN with exception of 2-(p-methoxyphenyl)-5-methyl-cyclohexanone and 2-(p-methoxyphenyl)-4-methyl-1,3-dioxane.

2. Process for the preparation of cyclohexane derivatives of the formula I according to Claim 1, characterized in that a compound which otherwise corresponds to the formula I, but contains one or more reducible groups and/or C - C bonds instead of H atoms is treated with a reducing agent, or,

in order to prepare esters of the formula I, a corresponding carboxylic acid or one of its reactive derivatives is reacted with a corresponding alcohol or one of its reactive derivatives, or,

in order to prepare β-keto esters of the formula I, a corresponding cyclohexanone derivative is subjected to a condensation reaction with a corresponding dialkyl carbonate in the presence of a base, or,

in order to prepare ethers of the formula I, a corresponding hydroxy compound is etherified, or, in order to prepare cyclohexanone derivatives of the formula I, a corresponding epoxide is subjected to an acid-catalysed rearrangement.

3. Use of the compounds of the formula I according to Claim 1 as components of liquid-crystal phases.

4. Liquid-crystal phase having at least two liquid-crystal components, characterized in that it contains at least one transversely polarized 1,4-cyclohexylene compound wherein at least one $CH_2$ group has been replaced by a -CO- group.

5. Liquid-crystal phase having at least two liquid-crystal components, characterized in that it contains at least one cis-1,3-disubstituted cyclohexane compound.

6. Liquid-crystal phase according to Claim 4 or 5, characterized in that at least one component is a compound of the formula I according to Claim 1.

7. Liquid-crystal display element characterized in that it contains a phase according to Claim 4, 5 or 6.

8. Electrooptical display element according to Claim 7, characterized in that it contains, as the dielectric, a phase according to Claim 4, 5 or 6.

## Revendications

1. Dérivés de cyclohexane de formule I

$R^1$-$A^1$-$Z^1$-$A^2$-$R^2$ I

dans laquelle

$R^1$ et $R^2$ représentent chacun H, un groupe alkyle ayant de 1 à 10 atomes de carbone dans lequel un ou deux

groupes $CH_2$ non voisins peuvent aussi être remplacés par des atomes d'oxygène et/ou par des groupes -CO- et/ou par des groupes -CO-O- et/ou par des groupe -CH=CH-, F, Cl, Br, CN ou $R^3$-$A^3$-$Z^2$-,

$A^1$ est -A-, $A^4$-$Z^o$-A- ou -A-$Z^o$-$A^4$-,

A est un groupe trans-1,4-cyclohexylène ou 1,4-cyclohexénylène, non substitué ou substitué une ou plusieurs fois, en position 2, 3, 5 et/ou 6, par F et/ou Cl et/ou par Br et/ou par CN et/ou par un groupe alkyle ou un groupe alkyle fluoré ayant chacun de 1 à 10 atomes de carbone, où un ou deux groupes $CH_2$ non voisins peuvent aussi être remplacés par des atomes d'oxygène et/ou par des groupes -CO- et/ou par des groupes -CO-O-, et pouvant éventuellement aussi être substitué en position 1 et/ou en position 4, auquel cas un ou deux groupes $CH_2$ sont remplacés par le radical -CO-, ou bien

$R^1$-$A^1$- représente un groupe cyclohexyle substitué en position 3 par un groupe alkyle ayant de 1 à 10 atomes de carbone dans lequel un ou deux groupes $CH_2$ non voisins peuvunt aussi être remplacés par des atomes d'oxygène et/ou par des groupes -CO- et/ou par des groupes -CO-O- et/ou par des groupes -CH=CH-, ou par -CN auquel cas un ou deux groupes $CH_2$ non voisins peuvent être remplacés par -O- ou -CO-,

$A^2$, $A^3$ et $A^4$ représentent chacun un radical 1,4-phénylène, non substitué ou substitué par 1 ou 2 atomes de fluor et/ou de chlore et/ou par des groupes $CH_3$ et/ou par des groupes CN, dans lequel un ou deux groupes CH peuvent aussi être remplacés par des atomes d'azote et/ou par NO; un radical 1,4-cyclohexylène, où un ou deux groupes $CH_2$ non voisins peuvent aussi être remplacés par des atomes d'oxygène; les groupes 1,3-dithian-2,5-diyle, pipéridine-1,4-diyle, 1,4-bicyclo(2,2,2)-octylène, décahydronaphtalène-2,6-diyle, ou 1,2,3,4-tétrahydronaphtalène-2,6-diyle, ou encore -A-;

$Z^o$, $Z^1$ et $Z^2$ représente, chacun, -CO-O-, -O-CO-, -$CH_2CH_2$-, -$OCH_2$-, -$CH_2O$-, -$CH_2CO$-, -$COCH_2$-, -$CH_2CHCN$-, -$CHCNCH_2$- ou une simple liaison, et

$R^3$ est H, un groupe alkyle ayant de 1 à 10 atomes de carbone, dans lequel un ou deux groupes $CH^2$ non voisins peuvent aussi être remplacés par des atomes d'oxygène et/ou par des groupes -CO- et/ou par des groupes -CO-O- et/ou par des groupes -CH=CH-, F, Cl, Br ou CN,

à l'exception de la 2-(méthoxyphényl)-5-méthylcyclo-hexanone et du 2-(p-méthoxyphényl)-4-méthyl-1,3-dioxanne.

2. Procédé pour la préparation de dérivés de cyclohexane de formule I selon la revendication 1, caractérisé en ce qu'on traite avec un agent réducteur un composé correspondant pour le reste à la formule I mais contenant à la place des atomes H un ou plusieurs groupes réducteurs et/ou des liaisons C - C,

ou encore que, pour préparer des esters de formule I, on fait réagir un acide carboxylique correspondant ou l'un de ses dérivés réactifs avec un alcool correspondant ou l'un de ses dérivés réactifs,

ou bien que, pour préparer des β-cétoesters de formule I, on condense un dérivé correspondant de cyclohexanone, en présence d'une base, avec un carbonate de dialkyle correspondant,

ou bien que, pour préparer des éthers de formule I, on éthérifie un composé hydroxy correspondant,

ou bien que, pour préparer des dérivés de cyclohexanone de formule I, on transpose un époxyde correspondant, par catalyse acide.

3. Utilisation des composés de formule I selon la revendication 1, comme composants de phases à cristaux liquides.

4. Phase à cristaux liquides comportant au moins deux composants à cristaux liquides, caractérisée en ce qu'elle contient au moins un composé 1,4-cyclohexylène à polarisation croisée, où au moins un groupe $CH_2$ est remplacé par un groupe -CO.

5. Phase à cristaux liquides comportant au moins deux constituants à cristaux liquides, caractérisée en ce qu'elle contient au moins un composé du cyclohexane cis-1,3-disubstitué.

6. Phase à cristaux liquides selon les revendications 4 ou 5, caractérisée en ce qu'au moins un constituant est un composant de fomule I selon la revendication 1.

7. Elément d'affichage à cristaux liquides, caractérisé en ce qu'il contient une phase selon les revendications 4, 5 ou 6.

8. Elément d'affichage électrooptique selon la revendication 7, caractérisé en ce qu'il contient comme diélectrique une phase selon les revendications 4, 5 ou 6.